# EUROPÄISCHE PATENTSCHRIFT

(11) **EP 3 227 023 B1**
(45) Veröffentlichungstag und Bekanntmachung des Hinweises auf die Patenterteilung: **25.03.2020**
(21) Anmeldenummer: 15763938.6
(22) Anmeldetag: 18.09.2015
(51) Int. Cl.: B01L 3/00, B01F 13/00, B01F 15/02, B01F 3/08, C12Q 1/6806, B01F 5/06

(54) **VERFAHREN ZUR TROPFENERZEUGUNG**
METHOD FOR PRODUCING DROPS
PROCÉDÉ DE PRODUCTION DE GOUTTES

(30) Priorität: 02.12.2014 DE 102014224664
(43) Veröffentlichungstag der Anmeldung: 11.10.2017
(73) Patentinhaber: Hahn-Schickard-Gesellschaft für angewandte Forschung e.V., 78052 Villingen-Schwenningen (DE)
(72) Erfinder: SCHWEMMER, Frank, 79106 Freiburg (DE); SCHULER, Friedrich, 53639 Königswinter (DE)
(74) Vertreter: Stöckeler, Ferdinand
(86) Internationale Anmeldenummer: PCT/EP2015/071435
(87) Internationale Veröffentlichungsnummer: WO 2016/087068

(56) Entgegenhaltungen:
- WO-A1-2013/120190
- WO-A1-2014/210207
- DE-A1- 10 361 411
- US-A1- 2006 094 119
- Suzanne Hugo ET AL: "A centrifugal microfluidic platform for point-of-care diagnostic applications", South African Journal of Science, 1. Januar 2014 (2014-01-01), Seiten 1-7, XP055229282, DOI: 10.1590/sajs.2014/20130091 Gefunden im Internet: URL:http://www.scielo.org.za/pdf/sajs/v110 n1-2/13.pdf [gefunden am 2015-11-18]

## Beschreibung

Die vorliegende Erfindung bezieht sich auf Verfahren zur Erzeugung von Tropfen einer ersten Flüssigkeit in einer zweiten Flüssigkeit und insbesondere Verfahren zur Tropfenerzeugung in der zentrifugalen Mikrofluidik.

Die zentrifugale Mikrofluidik beschäftigt sich mit der Handhabung von Flüssigkeiten im Femtoliter- bis Milliliter-Bereich in rotierenden Systemen. Solche Systeme sind meist Polymer-Einwegkartuschen, die in oder anstelle von Zentrifugenrotoren verwendet werden, mit der Absicht Laborprozesse zu automatisieren. Dabei können Standardlaborprozesse, wie Pipettieren, Zentrifugieren, Mischen oder Aliquotieren in einer mikrofluidischen Kartusche implementiert werden. Zu diesem Zweck beinhalten die Kartuschen Kanäle für die Fluidführung, sowie Kammern für das Auffangen von Flüssigkeiten. Die Kartuschen werden mit einer vordefinierten Abfolge von Drehfrequenzen, dem sogenannten Frequenzprotokoll, beaufschlagt, so dass die in den Kartuschen befindlichen Flüssigkeiten durch die Zentrifugalkraft bewegt werden können.

Anwendung findet die zentrifugale Mikrofluidik hauptsächlich in der Laboranalytik und in der mobilen Diagnostik. Solche Kartuschen können als zentrifugal-mikrofluidische Scheiben ausgeführt sein, wie sie unter den Bezeichnung "Lab-on-a-disk", "LabDisk", und "Lab-on-CD", etc., bekannt sind, die in speziellen Prozessiergeräten eingesetzt werden. Andere Formate, wie mikrofluidische Zentrifugenröhrchen, die beispielsweise unter der Bezeichnung "LabTube" bekannt sind, können in Rotoren bereits bestehender Standardlaborgeräte eingesetzt werden.

Eine wesentliche Grundoperation, die in zentrifugal-mikrofluidischen Kartuschen ausgeführt werden muss, ist das gezielte Aliquotieren eines Flüssigkeitsvolumens in verschiedene Subvolumina, sogenannte Aliquots. Für die Verwendung dieser Grundoperation in einem möglichen Produkt ist die Robustheit und Einfachheit der Handhabung des Prozesses von höchster Bedeutung. Ferner sollte die Grundoperation monolithisch realisiert sein, so dass keine zusätzlichen Komponenten oder Materialien, die durch Materialkosten oder zusätzliche Aufbau- und Verbindungstechnik (Assemblierung) die Kosten der Kartusche wesentlich steigern, erforderlich sind.

Verschiedene Anwendungen, zum Beispiel die digitale PCR (Polymerase-Kettenreaktion), Einzelzellverfahren, das Zählen von Bakterien mittels fluoreszenter Phagen und die Herstellung von Partikeln im Mikrometerbereich, erfordern die Herstellung von sehr vielen Aliquots. Es müssen Stückzahlen von einigen hundert bis über einer Million Aliquots hergestellt werden
Für viele Anwendungen ist es wichtig, Aliquots kleiner Größe (wenige Mikroliter bis Pikoliter oder Femtoliter) zu erstellen. Das ist vor allem dann von hoher Wichtigkeit, wenn eine gewisse Menge an Aliquots erzeugt werden muss, um ein gewünschtes Experiment durchzuführen, das Ausgangsvolumen aber limitiert ist, wie z.B. bei der Digital-PCR. Häufig stellen hohe Kosten für Reagenzien, teure Aufreinigung von Probenmaterial oder geringe Mengen von Probenmaterial eine Limitierung für solche Anwendungen dar.

Es existiert somit der Bedarf einer Grundoperation für zentrifugale, mikrofluidische Systeme, die ein gezieltes Aliquotieren eines Volumens in viele Aliquots (einige hundert bis über eine Million) kleinen Volumens (wenige Mikroliter bis Femtoliter) ermöglichen. Es ist eine Vielzahl von Techniken für die Erzeugung von Tröpfchen auf druckgetriebenen mikrofluidischen und zentrifugalen mikrofluidischen Plattformen bekannt.

Dokument DE10361411 bezieht sich auf eine Mischervorrichtung und ein Verfahren zum Mischen von zumindest zwei Flüssigkeiten.

Dokument WO2013/120190 bezieht sich auf ein Verfahren zur Mischung zweier Fluide in einer mikrofluidischen Vorrichtung.

Bekannte druckgetriebene Verfahren zur Erzeugung von Tropfen einer wässrigen Lösung in Öl verwenden ein Mikrokanalsystem, um die wässrige Lösung in Öl zu emulgieren. Dabei fließt die wässrige Phase durch einen Kanal in eine ölgefüllte Kammer. Sie verdrängt das Öl und fließt eine Stufe hinauf auf ein Plateau. Dieses Plateau ist durch eine Reihe von Wänden in Kanäle unterteilt. Die wässrige Phase durchfließt diese Kanäle auf das dahinterliegende Plateau. Von dort fließt die Phase in eine in Flussrichtung dahinter liegende Kammer und erzeugt durch Abriss von Tropfen an der Kante zur Kammer eine Emulsion. Solche Verfahren sind beispielsweise bei [6], [9] bis [20] und [22] beschrieben.

Bei [8] ist ein Verfahren beschrieben, bei dem eine druckgetriebene Erzeugung und ein Transport von Gasblasen in Flüssigkeiten durch eine sich ändernde Kammerhöhe erfolgt. Solche Verfahren ermöglichen die Erzeugung von Blasen einer gasförmigen Phase in einer flüssigen Phase mit Hilfe eines Mikrokanalsystems. Dabei fließt die gasförmige Phase durch einen Kanal in eine Kammer, die mit der wässrigen Phase gefüllt ist. Die Kammer ist so abgeschrägt, dass sich ihr flaches Ende an der Mündung zum Kanal befindet und genauso hoch ist wie die Höhe des Kanals. Angetrieben durch einen Druck fließt die gasförmige Phase an die Mündungsstelle des Kanals, wo sich eine Blase in die zweite Phase schiebt. Bedingt durch die sich aufweitende Kammer reißen Blasen definierter Größe von der Flüssigkeitszunge ab und wandern in Flussrichtung in die Kammer hinein, getrieben durch die Aufweitung der Kammerhöhe und Kapillarkräfte.

[1] und [2] beschreiben ein Verfahren zur druckgetriebenen Erzeugung und zum Transport von Flüssigkeitstropfen in Flüssigkeiten durch eine sich ändernde Kammerhöhe. Dieses Verfahren ermöglicht die Erzeugung von Tropfen einer ersten flüssigen Phase in einer zweiten flüssigen Phase mit Hilfe eines Mikrokanalsystems. Dabei fließt die erste Phase durch einen Kanal in eine Kammer, die mit der zweiten Phase gefüllt ist. Die Kammer ist so abgeschrägt, dass sich ihr flaches Ende an der Mündung zum Kanal befindet und genauso hoch ist wie die Höhe des Kanals. Angetrieben durch eine Pumpe fließt die erste Phase an die Mündungsstelle des Kanals, wo sich eine Flüssigkeitszunge in die zweite Phase schiebt. Bedingt durch die sich aufweitende Kammer reißen Tropfen definierter Größe von der Flüssigkeitszunge ab und wandern in Flussrichtung in die Kammer hinein, getrieben durch die Aufweitung der Kammerhöhe und Kapillarkräfte.

Ein vergleichbares Verfahren ist in [23] beschrieben. Ein druckbetriebenes System, also kein zentrifugales, das ein Gerät zur Erzeugung von Tropfen aufweist, ist beschrieben. Kernbestandteil ist eine Aufweitung zur Erzeugung von Tropfen. Nach einer ersten Befüllung mit z.B. Öl wird eine zweite Phase, z.B. Wasser, durch Kapillarkräfte an der Aufweitung emulgiert. Die Größe der Tropfen wird hauptsächlich durch die Geometrie der Aufweitung bestimmt. Ferner ist eine Parallelisierung durch eine kreisförmige Anordnung beschrieben.

[3] und [7] offenbaren ein Verfahren zur zentrifugalen Erzeugung von Flüssigkeitstropfen in Luft. Dieses Verfahren ermöglicht die Erzeugung von Flüssigkeitstropfen in Luft mit Hilfe eines Mikrokanalsystems und daran anschließend das Auffangen der Tropfen in einer wässrigen Lösung. Dabei fließt die erste flüssige Phase angetrieben durch Zentrifugalkraft durch einen Kanal in eine Kapillare, an deren Ende sich eine Mikrodüse befindet, die frei in der Luft hängt. Am Ende der Kapillare reißen ab einer gewissen Frequenz Tropfen ab, die eine kurze Strecke durch die umgebende Luft fliegen und dann auf die Oberfläche einer Flüssigkeit in einem Sammelgefäß treffen. Dort härten die Tropfen durch eine biochemische Reaktion (teilweise) aus und werden gesammelt. Dabei ist das Auffanggefäß so angebracht, dass es in Ruhe relativ zum Erdboden senkrecht befindet und erst durch Ausüben einer Zentrifugalkraft in die Waagerechte gebracht wird.

Bei [5] ist ein Verfahren zur zentrifugalen Erzeugung von abgeschlossenen Flüssigkeitsvolumen auf einer rotierenden Scheibe beschrieben. Dieses Verfahren ermöglicht die Erzeugung von abgeschlossenen Flüssigkeitsvolumina mit Hilfe eines Mikrokanal- und Mikrovertiefungs-Systems. Eine erste Flüssigkeit wird in eine Einlasskammer eines mikrofluidischen Systems auf einer rotierenden Scheibe gebracht. Durch Zentrifugalkraft bewegt sich diese Flüssigkeit in eine Kammer mit vielen kleinen Vertiefungen, die sich mit der ersten Flüssigkeit füllen. Eine zweite unmischbare Flüssigkeit wird genutzt, um den Überstand der ersten Flüssigkeit oberhalb der Wände der Vertiefungen zu verdrängen. Dadurch wird der direkte Kontakt der Flüssigkeitsvolumina der ersten Flüssigkeit in den Vertiefungen untereinander unterbrochen.

In [4] und [21] sind Vorrichtungen und Verfahren zum Erzeugen eines Gemenges von zwei ineinander unlösbaren Phasen beschrieben. Eine zentrifugal mikrofluidische Scheibe zur Erzeugung von Tropfen ist vorgesehen, wobei die Tropfenerzeugung auf dem Prinzip eines Mantelflusses basiert. Ein Tropfenabriss einer wässrigen Phase aus einem ersten Kanal wird durch Abschnüren durch einen Fluss von Öl aus benachbarten Kanälen erzeugt. Nach der Mündung der benachbarten Kanäle in den ersten Kanal weitet sich der erste Kanal auf und die erzeugten Tropfen gelangen in den aufgeweiteten Abschnitt des ersten Kanals.

Der vorliegenden Erfindung liegt die Aufgabe zugrunde, ein alternatives Verfahren zu schaffen, das es ermöglicht zentrifugal einen oder mehrere Tropfen einer Flüssigkeit zu erzeugen, der oder die in eine andere Flüssigkeit eingebettet sind.

Diese Aufgabe wird durch ein Verfahren nach Anspruch 1 gelöst.

Ausführungsbeispiele der Offenbarung werden nachfolgend Bezug nehmend auf die beiliegenden Zeichnungen näher erläutert. Es zeigen:
Fig. 1 schematisch Fluidikstrukturen, bei denen ein Fluidkanal in einem radial äußeren Abschnitt in eine Fluidkammer mündet;
Fig. 2 schematisch Fluidikstrukturen, bei denen ein Fluidkanal in einem radial inneren Abschnitt in eine Fluidkammer mündet;
Fig. 3a bis 3c schematische Darstellungen zur Erläuterung von Übergangsbereichen;
Fig. 4 schematische Darstellungen, die eine Tropfenerzeugung an einem Übergangsbereich zeigen;
Fig. 5a bis 5c Diagramme unterschiedlicher Tropfengrößen bei Parameteränderungen des Fluidkanals und des Übergangsbereichs;
Fig. 6 eine schematische Darstellung einer Vorrichtung zur Tropfenerzeugung;
Fig. 7 schematische Darstellung von erzeugten Emulsionen;
Fig. 8 eine schematische Darstellung von Fluidikstrukturen zur Tropfenerzeugung kombiniert mit anderen Operationen;
Fig. 9a und 9b schematische Darstellungen von Fluidikstrukturen zur parallelen Erzeugung mehrerer Tropfen;
Fig. 10a und 10b schematische Darstellungen von Fluidikstrukturen zur parallelen Erzeugung mehrerer Tropfen mit unterschiedlichen Eigenschaften;
Fig. 11 eine schematische Darstellung alternativer Ausführungsbeispiele von Fluidikstrukturen; und
Fig. 12 und 13 schematische Seitenansichten zur Erläuterung von Ausführungsbeispielen von Vorrichtungen zur Tropfenerzeugung von einem oder mehreren Tropfen.

Bevor Ausführungsbeispiele der Erfindung näher erläutert werden, sei zunächst darauf hingewiesen, dass Beispiele der Offenbarung insbesondere auf dem Gebiet der zentrifugalen Mikrofluidik Anwendung finden können, bei der es um die Prozessierung von Flüssigkeiten im Femtoliter- bis Milliliter-Bereich geht. Entsprechend können die Fluidikstrukturen geeignete Abmessungen im Mikrometerbereich für die Handhabung entsprechender Flüssigkeitsvolumina aufweisen. Insbesondere können Ausführungsbeispiele der Offenbarung auf zentrifugal-mikrofluidischen Systemen Anwendung finden, wie sie beispielsweise unter der Bezeichnung "Lab-on-a-Disk" bekannt sind.

Wird hierin der Ausdruck radial verwendet, so ist jeweils radial bezüglich eines Rotationszentrums, um der Rotationskörper drehbar ist, gemeint. Im Zentrifugalfeld ist somit eine radiale Richtung von dem Rotationszentrum weg radial abfallend und eine radiale Richtung zu dem Rotationszentrum hin ist radial ansteigend. Ein Fluidkanal, dessen Anfang näher am Rotationszentrum liegt als dessen Ende, ist somit radial abfallend, während ein Fluidkanal, dessen Anfang weiter vom Rotationszentrum entfernt ist als dessen Ende, radial ansteigend ist. Ein Kanal, der einen radial ansteigenden Abschnitt aufweist, weist also Richtungskomponenten auf, die radial ansteigen bzw. radial nach innen verlaufen. Es ist klar, dass ein solcher Kanal nicht exakt entlang einer radialen Linie verlaufen muss, sondern in einem Winkel zu der radialen Linie oder gebogen verlaufen kann.

Ist hierin von einem Fluidkanal die Rede, so ist eine Struktur gemeint, deren Längenabmessung von einem Fluideinlass zu einem Fluidauslass größer ist, beispielsweise mehr als 5-mal oder mehr als 10-mal größer, als die Abmessung bzw. Abmessungen, die den Strömungsquerschnitt definiert bzw. definieren. Somit kann ein Fluidkanal einen Strömungswiderstand für ein Durchströmen desselben von dem Fluideinlass zu dem Fluidauslass aufweisen. Dagegen ist eine Fluidkammer hierein eine Kammer die solche Abmessungen aufweisen kann, dass ein relevanter Strömungswiderstand in derselben nicht auftritt.

Unter den Ausdruck Flüssigkeit bzw. flüssigen Phase, wie er hierin verwendet wird, fallen, wie Fachleuten offensichtlich ist, auch Flüssigkeiten, die Feststoffbestandteile beinhalten, wie z.B. Suspensionen und biologische Proben.

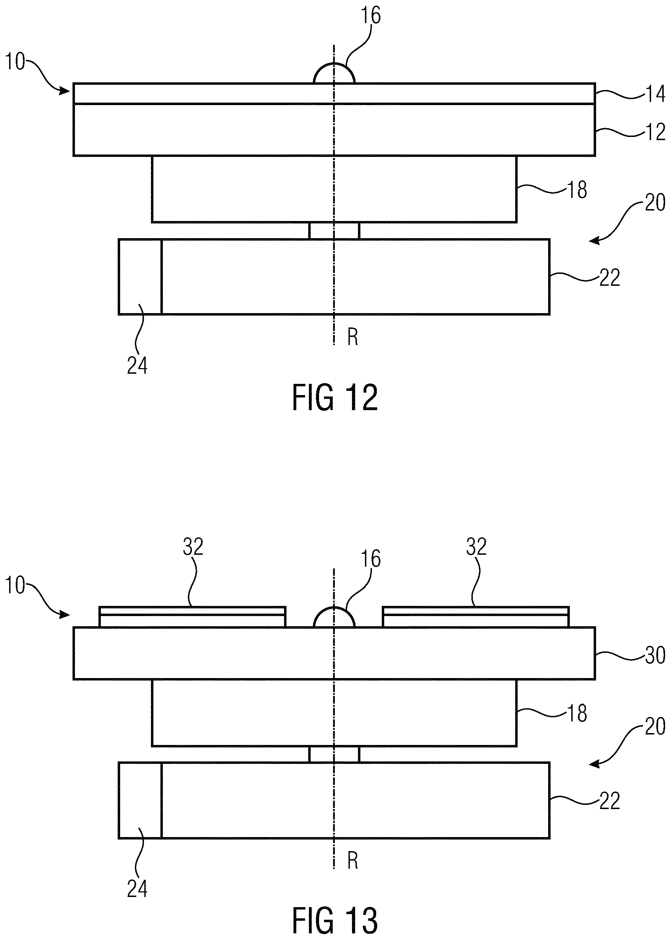

Bezug nehmend auf die Fig. 12 und 13 werden zunächst Beispiele von zentrifugal-mikrofluidischen Systemen beschrieben.

Fig. 12 zeigt eine Vorrichtung mit einem Fluidikmodul 10 in Form eines Rotationskörpers, der ein Substrat 12 und einen Deckel 14 aufweist. Das Substrat 12 und der Deckel 14 können in Draufsicht kreisförmig sein, mit einer mittigen Öffnung, über die der Rotationskörper 10 über eine übliche Befestigungseinrichtung 16 an einem rotierenden Teil 18 einer Antriebsvorrichtung 20 angebracht sein kann. Das rotierende Teil 18 ist drehbar an einem stationären Teil 22 der Antriebsvorrichtung 20 gelagert. Bei der Antriebsvorrichtung 20 kann es beispielsweise um eine herkömmliche Zentrifuge, die eine einstellbare Drehgeschwindigkeit aufweisen kann, oder auch ein CD- oder DVD-Laufwerk handeln. Eine Steuereinrichtung 24 kann vorgesehen sein, die ausgelegt ist, um die Antriebsvorrichtung 20 zu steuern, um den Rotationskörper 10 mit einer Rotation oder mit Rotationen unterschiedlichen Drehfrequenzen zu beaufschlagen. Die Steuereinrichtung 24 kann, wie für Fachleute offensichtlich ist, beispielsweise durch eine entsprechend programmierte Recheneinrichtung oder eine anwenderspezifische integrierte Schaltung implementiert sein. Die Steuereinrichtung 24 kann ferner ausgelegt sein, um auf manuelle Eingaben durch einen Benutzer hin die Antriebsvorrichtung 20 zu steuern, um die erforderlichen Rotationen des Rotationskörpers zu bewirken. In jedem Fall kann die Steuereinrichtung 24 konfiguriert sein, um die Antriebsvorrichtung 20 zu steuern, um den Rotationskörper mit der erforderlichen Rotation zu beaufschlagen. Als Antriebsvorrichtung 20 kann eine herkömmliche Zentrifuge mit nur einer Drehrichtung verwendet werden.

Der Rotationskörper 10 weist die erforderlichen Fluidikstrukturen auf. Die erforderlichen Fluidikstrukturen können durch Kavitäten und Kanäle in dem Deckel 14, dem Substrat 12 oder in dem Substrat 12 und dem Deckel 14 gebildet sein. Bei Ausführungsbeispielen können beispielsweise Fluidikstrukturen in dem Substrat 12 abgebildet sein, während Einfüllöffnungen und Entlüftungsöffnungen in dem Deckel 14 gebildet sind. Bei Ausführungsbeispielen ist das strukturierte Substrat (inklusive Einfüllöffnungen und Entlüftungsöffnungen) oben angeordnet und der Deckel unten angeordnet.

Bei einem alternativen in Fig. 13 gezeigten Ausführungsbeispiel sind Fluidikmodule 32 in einen Rotor 30 eingesetzt und bilden zusammen mit dem Rotor 30 den Rotationskörper 10. Die Fluidikmodule 32 können jeweils ein Substrat und einen Deckel aufweisen, in denen wiederum entsprechende Fluidikstrukturen gebildet sein können. Der durch den Rotor 30 und die Fluidikmodule 32 gebildete Rotationskörper 10 ist wiederum durch eine Antriebsvorrichtung 20, die durch die Steuereinrichtung 24 gesteuert wird, mit einer Rotation beaufschlagbar.

In den Figuren 12 und 13 ist ein Rotationszentrum, um das das Fluidikmodul bzw. der Rotationskörper drehbar ist, mit R bezeichnet.

Bei Ausführungsbeispielen der Offenbarung können das Fluidikmodul bzw. der Rotationskörper, das bzw. der die Fluidikstrukturen aufweist, aus einem beliebigen geeigneten Material gebildet sein, beispielsweise einem Kunststoff, wie PMMA (Polymethylmethacrylat), PC (Polycarbonat), PVC (Polyvinylchlorid) oder PDMS (Polydimethylsiloxan), Glas oder dergleichen. Der Rotationskörper 10 kann als eine zentrifugal-mikrofluidische Plattform betrachtet werden. Bei bevorzugten Ausführungsbeispielen können das Fluidikmodul bzw. der Rotationskörper aus einem Thermoplast, wie z.B. PP (Polypropylen), PC, COP (Cyclic Olefin Polymer), COC (Cyclo Olefin Copolymer) oder PS (Polystyrol) gebildet sein.

Nachfolgend werden Bezug nehmend auf die Figuren Ausführungsbeispiele von Fluidikstrukturen, die in einem entsprechenden Fluidikmodul 32 bzw. in einem entsprechenden Rotationskörper 10 gebildet sein können, beschrieben.

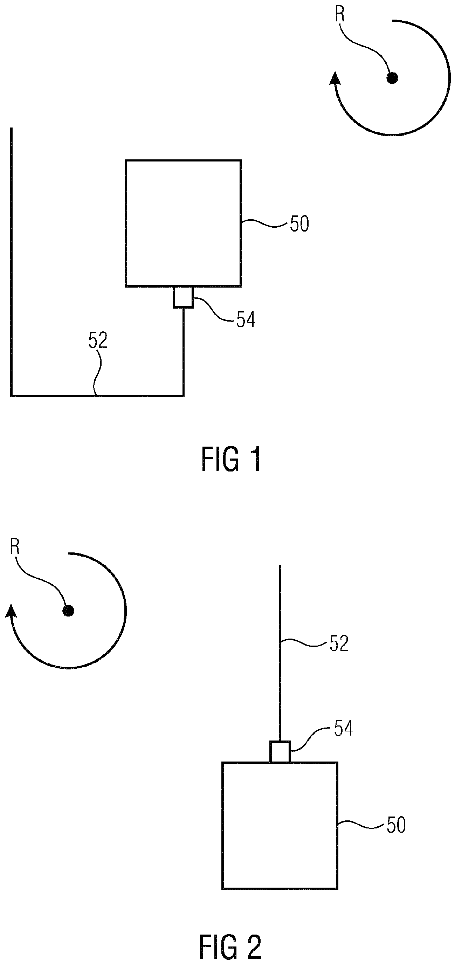

Wie in den Figuren 1 und 2 gezeigt ist, weisen die Fluidikstrukturen eine Fluidkammer 50, einen Fluidkanal 52 und einen Übergangsbereich 54 auf. Der Rotationskörper und damit die Fluidstrukturen sind um ein Rotationszentrum R drehbar. Die Fluidkammer 50 ist ausgebildet, um eine Flüssigkeit, die hierin auch als zweite Flüssigkeit bezeichnet wird, aufzunehmen. Die zweite Flüssigkeit kann beispielsweise Öl sein. Der Fluidkanal 52 ist ausgebildet, um eine Flüssigkeit, die hierin auch als erste Flüssigkeit bezeichnet wird, durch einen durch eine Rotation des Rotationskörpers bedingten hydrostatischen Zentrifugaldruck zu dem Übergangsbereich 54 und somit zu der Fluidkammer 50 zuzuführen. Die erste Flüssigkeit kann beispielsweise eine wässrige Lösung sein. Die Offenbarung ist jedoch nicht auf solche Flüssigkeiten beschränkt, sondern kann unter Verwendung anderer Flüssigkeiten implementiert werden, solange die erste und die zweite Flüssigkeit, die vorzugsweise unterschiedliche Dichten aufweisen, nicht mischbar sind.

Der Übergangsbereich 54 zwischen dem Fluidkanal 52 und der Fluidkammer 50 ist so gestaltet, dass eine Strömung der ersten Flüssigkeit, die in der zweiten Flüssigkeit nicht mischbar ist, durch den Fluidkanal (52) in Richtung der Fluidkammer, hervorgerufen durch Rotation des Substrates und einen dadurch entstehenden hydrostatischen Zentrifugaldruck, das Entstehen von Tropfen der ersten Flüssigkeit eingebettet in der zweiten Flüssigkeit hervorruft. Hierbei fließt nur die erste Flüssigkeit signifikant. Die in den Figuren 1 und 2 gezeigten Ausführungsbeispiele der Offenbarung enthalten keine weiteren Kanäle, wobei sowohl die Fluidkammer 50 als auch der Fluidkanal 52 belüftet sein können.

Bei dem in Fig. 1 gezeigten Ausführungsbeispiel mündet der Fluidkanal 52 in einem radial äußeren Bereich in die Fluidkammer 50. Dieses Ausführungsbeispiel ist dafür ausgelegt, dass die Dichte der ersten Flüssigkeit geringer ist als die Dichte der zweiten Flüssigkeit, d.h., dass die emulgierte Phase leichter ist als die umgebende Phase. Aufgrund des durch die Rotation bewirkten Zentrifugalfelds steigen bei diesem Ausführungsbeispiel die erzeugten leichteren Tropfen der ersten Flüssigkeit in der Fluidkammer 50 radial nach innen und bewegen sich von dem Übergangsbereich weg. Somit kann der Auftrieb des Zentrifugalfelds genutzt werden, um die leichteren Tropfen von dem Ort der Erzeugung derselben weg zu bewegen und die zweite Flüssigkeit an diesem Ort zu halten.

Ein Vorteil zentrifugaler Tropfenerzeugung besteht insbesondere darin, dass bei Ausführungsbeispielen, bei denen das kontinuierliche Medium, also die zweite Flüssigkeit wie z.B. Öl, dichter ist als die erste Flüssigkeit, wie z.B. Wasser, das kontinuierliche Medium durch die Zentrifugalkräfte an der Aufweitung gehalten wird. Das ist insbesondere dann von Vorteil, wenn man versucht, Emulsionen herzustellen, bei denen möglichst viele Tropfen in möglichst wenig kontinuierlicher Phase enthalten sind. Der Stand der Technik erwähnt dabei ein Verhältnis von 96% Tropfenvolumen und 4% Volumen der kontinuierlichen Phase. Mit Hilfe von Ausführungsbeispielen der Offenbarung ist es möglich, dieses Verhältnis deutlich zu verbessern, nämlich beispielsweise auf 97,2% Tropfenvolumen und 2,8% Volumen der kontinuierlichen Phase. Das entspricht einer Einsparung von 30% der kontinuierlichen Phase und ermöglicht die Herstellung von Gelemulsionen in situ.

Bei dem in Fig. 2 gezeigten Ausführungsbeispiel mündet der Fluidkanal 52 in einem radial inneren Bereich in die Fluidkammer. Dieses Ausführungsbeispiel ist dafür ausgelegt, dass die Dichte der ersten Flüssigkeit größer ist als die Dichte der zweiten Flüssigkeit, d.h. die emulgierte Phase ist schwerer als die umgebende Phase. Aufgrund des durch die Rotation bewirkten Zentrifugalfelds werden bei diesem Ausführungsbeispiel die erzeugten schwereren Tropfen der ersten Flüssigkeit in der Fluidkammer 50 radial nach außen getrieben und bewegen sich von dem Übergangsbereich weg.

Abweichend zu den in den Figuren 1 und 2 gezeigten Ausführungsbeispielen, bei denen das Rotationszentrum oberhalb der Fluidikstrukturen gezeigt ist, könnte das Rotationszentrum auch unterhalb der Fluidikstrukturen angeordnet sein, was zu einem geraden Kanal, der in ein radial äußeres Ende der Fluidkammer mündet, und einem gewinkelten Kanal, der in ein radial inneres Ende der Fluidkammer mündet, führen würde.

Durch eine Rotation des Rotationkörpers kann somit die erste Flüssigkeit, kontrolliert in die zweite Flüssigkeit emulgiert werden. Dadurch kann ein Tropfen der ersten Flüssigkeit in der zweiten Flüssigkeit oder eine Mehrzahl von Tropfen der ersten Flüssigkeit in der zweiten Flüssigkeit eingebettet werden, wobei die Anzahl der Tropfen von der Dauer der Rotation abhängen kann. Bei Ausführungsbeispielen der Offenbarung kann ein gesamtes Volumen der ersten Flüssigkeit, das über den Fluidkanal zugeführt wird, in eine große Zahl von Tropfen aufgeteilt werden, die in die zweiten Flüssigkeit eingebettet sind.

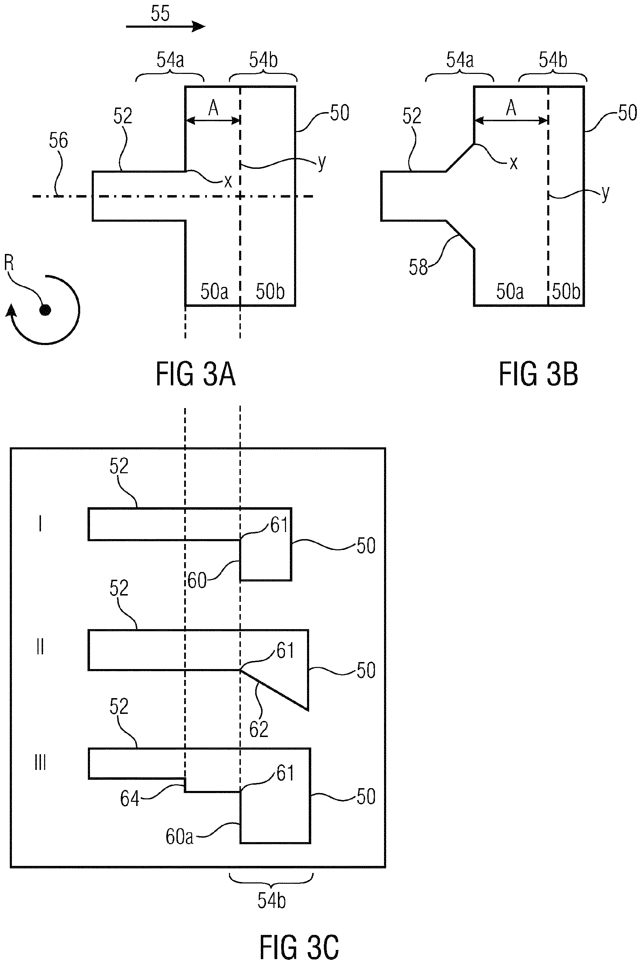

Bezugnehmend auf die Fig. 3a bis 3c werden nun Ausführungsbeispiele des Übergangsbereichs näher erläutert. Dabei stellen die Figuren 3a und 3 schematische Draufsichten auf jeweilige Übergangsbereiche 54 dar und Fig. 3c stellt schematische Längsschnittansichten jeweiliger Übergangsbereiche 54 dar (wobei die Längsschnitte für beide in den Figuren 3a und 3b gezeigten Strukturen gelten können). Eine Strömungsrichtung ist in den Figuren 3a bis 3c von links nach rechts und ist durch einen Pfeil 55 dargestellt.

Der Übergangsbereich 54 weist einen ersten Aufweitungsbereich 54a auf, in dem sich der Strömungsquerschnitt für den Fluss der ersten Flüssigkeit in einer ersten Richtung senkrecht zu der Strömungsrichtung 55 aufweitet. Die erste Richtung kann beispielsweise der Breitenrichtung des Fluidkanals entsprechen. Mit anderen Worten weitet sich der Fluidkanal in dem ersten Aufweitungsbereich 54a in einer ersten Dimension auf. Diese Aufweitung kann plötzlich, also sprungförmig, erfolgen, siehe Fig. 3a, oder zumindest teilweise kontinuierlich, siehe Aufweitung 58 in Fig. 3b. Wie in den Fig. 3a und 3b zu erkennen ist, weitet sich der Strömungsquerschnitt vorzugsweise in zueinander entgegengesetzte erste Richtungen auf, d.h. nach links und rechts bezüglich einer gedachten Mittellinie 56 (längs) des Fluidkanals 52. Die Aufweitung in den zueinander entgegengesetzten ersten Richtungen kann symmetrisch erfolgen.

Der Übergangsbereich 54 weist ferner einen zweiten Aufweitungsbereich 54b auf, der stromabwärts von dem ersten Aufweitungsbereich 54a angeordnet ist, und in dem sich der Strömungsquerschnitt für den Fluss der ersten Flüssigkeit in einer zweiten Richtung, die senkrecht zu der ersten Richtung und der Strömungsrichtung ist, aufweitet. Die zweite Richtung kann beispielsweise die Höhenrichtung des Fluidkanals 52 sein. Mit anderen Worten weitet sich der Kanal im zweiten Aufweitungsbereich in einer zweiten Dimension auf. Die Aufweitung im zweiten Aufweitungsbereich ergibt definiert eine Kante 61 (siehe Fig. 3c), die sich vorzugsweise über die gesamte Breite der durch die Aufweitung im ersten Aufweitungsbereich erhaltene Struktur erstreckt.

Wie in den Figuren 3a und 3b durch einen Abstand A gezeigt ist, kann der Übergangsbereich einen Abschnitt gleichbleibenden Querschnitts aufweisen, in dem dem Fluss der ersten Flüssigkeit zwischen dem ersten Aufweitungsbereich 54a und dem zweiten Aufweitungsbereich 54b ein gleichbleibender Strömungsquerschnitt geboten wird. Dieser Bereich kann als Terrasse bezeichnet werden, da in diesem Bereich der Kammerboden verglichen mit dem Kammerboden nach dem zweiten Aufweitungsbereich erhöht ist.

Ausführungsbeispiele von Aufweitungen im zweiten Aufweitungsbereich 54b sind in den schematischen Längsschnittansichten I, II und III in Fig. 3c gezeigt. Bei I ist eine sprungförmige Aufweitung 60 gezeigt, bei II ist eine kontinuierliche Aufweitung 62 gezeigt und bei III ist eine sprunghafte Aufweitung 60a gezeigt. Ferner ist bei dem in III gezeigten Ausführungsbeispiel eine Aufweitung 64 in der zweiten Richtung in dem ersten Aufweitungsbereich 54a vorgesehen.

Allgemein weitet sich der Strömungsquerschnitt in dem ersten Aufweitungsbereich 54a in der ersten Richtung (bzw. den ersten entgegengesetzten Richtungen) auf, wobei gleichzeitig eine Aufweitung in anderen Richtungen stattfinden kann, beispielsweise der zweiten Richtung, wobei die Aufweitung in der anderen Richtung in der Regel geringer sein wird als die Aufweitung in der/den ersten Richtung(en). Allgemein weitet sich der Strömungsquerschnitt in dem zweiten Aufweitungsbereich 54b in der zweiten Richtung auf, wobei gleichzeitig eine Aufweitung in anderen Richtungen stattfinden kann. Es sind somit auch Fälle umfasst, bei denen der Fluidkanal in einem anderen als einem senkrechten Winkel in die Fluidkammer mündet.

Anders ausgedrückt mündet der Flusskanal 52 an einer Position X in die Fluidkammer 50, d.h. in einen Bereich 50a der Fluidkammer, der die Terrasse darstellt. Im Mündungsbereich weitet sich der Kanal plötzlich, Fig. 3a, oder kontinuierlich, Fig. 3b, in der ersten Dimension auf. Wie in Fig. 3c gezeigt ist, weist der Bereich 50a der Fluidkammer eine gleichbleibende Höhe auf. Somit bietet der Bereich 50a dem Fluss der ersten Flüssigkeit einen konstanten Strömungsquerschnitt. Ab einer Position Y in Strömungsrichtung nimmt die Höhe der Fluidkammer 50 an der Kante 61 zu, wodurch eine Aufweitung in der zweiten Richtung stattfindet, durch die ein zweiter Bereich 50b der Kammer gebildet wird. Wie in Fig. 3c dargestellt ist, kann diese Zunahme sprungförmig oder kontinuierlich erfolgen. Dadurch ist der zweite Aufweitungsbereich 54b implementiert. Es ist klar, dass in den Fig. 3a bis 3c nur die für den Übergangsbereich relevanten Abschnitte des Fluidkanals 52 und der Fluidkammer 50 gezeigt sind.

Mit anderen Worten trifft der Fluidkanal 52 auf die Fluidkammer 50. Der Fluidkanal 52 weitet sich in mindestens einer Dimension plötzlich oder kontinuierlich auf. Gleichzeitig kann sich der Fluidkanal 52 in einer zweiten Dimension plötzlich oder kontinuierlich aufweiten. Bevorzugt weitet sich der Fluidkanal 52 in der zweiten Dimension nicht gleichzeitig auf. Weitet sich der Fluidkanal gleichzeitig in der zweiten Dimension auf, so weitet er sich bevorzugt wenig auf verglichen mit der gleichzeitigen Aufweitung in der ersten Dimension. Die Fluidkammer 50 weitet sich von dem Ende, von der der Fluidkanal 52 zugeleitet wird zur anderen Seite hin auf. Diese Aufweitung kann plötzlich oder kontinuierlich erfolgen. Die Aufweitung beginnt nach einem Abstand A vom Übergang von Fluidkanal 52 zur Fluidkammer 50. Die Aufweitung erfolgt bevorzugt in einer Richtung senkrecht zur vorangegangenen Aufweitung des Fluidkanals bei der Mündung in die Fluidkammer. Unter bevorzugt ist dabei zu verstehen, dass die zusätzlich auch in andere Richtungen stattfinden kann.

Die Erfinder haben dabei erkannt, dass Fluidikstrukturen vergleichbar zu den Strukturen, wie sie für druckbasierte Systeme beispielsweise in [6], [9] bis [20] und [22] beschrieben sind, auf vorteilhafte Weise in einem zentrifugalen System bzw. einer zentrifugalen Plattform eingesetzt werden können.

Zur Erzeugung der Tropfen werden die Fluidkammer 50 und der Fluidkanal 52 mit einer zweiten flüssigen Phase, d.h. einer zweiten Flüssigkeit, gefüllt. Dies kann beispielsweise durch die Auswirkungen einer Zentrifugalkraft hervorgerufen durch eine Drehung der Fluidikstruktur um das Drehzentrum R bewirkt werden. Nachfolgend wird eine erste (weitgehend) flüssige Phase, die mit der ersten Phase nicht mischbar ist, über den Fluidkanal 52 einführt.

Die Strömung der ersten flüssigen Phase in Richtung der Fluidkammer 50 wird hervorgerufen durch eine Rotation des Substrats (d.h. des Rotationskörpers), z.B. auf Grund eines hydrostatischen Zentrifugaldrucks. Die Rotation kann mit einer konstanten Rotationsgeschwindigkeit erfolgen. Die Strömung der ersten in der zweiten unmischbaren flüssigen Phase durch die oben beschriebene Phase führt zu einem Tropfenabriss an der Aufweitung in der zweiten Dimension, d.h. der Aufweitung in dem zweiten Aufweitungsbereich 54b. Dabei ist das Volumen der erzeugten Tropfen im Wesentlichen durch die Geometrie der Aufweitung sowie die Oberflächenspannung und damit einhergehende Kapillarkräfte bestimmt. Die Tropfengröße ist weitestgehend unabhängig von der Flussrate der ersten Phase. Dabei fließt im wesentlich nur die erste flüssige Phase, während die zweite flüssige Phase im wesentlich ruht. Sowohl die Fluidkammer 50 als auch alle weiteren Strukturen wie zum Beispiel der Fluidkanal 50 können über einen Druckausgleich verfügen.

Der Durchmesser der erzeugten Tropfen ist dabei größer als die kleinste Kanaldimension des Übergangs. Das durch die Rotation des Rotationskörpers künstlich erzeugte Schwerefeld, welches am Übergang auf die Flüssigkeit wirkt, kann bei Ausführungsbeispielen mindestens der zweifachen Erdbeschleunigung entsprechen.

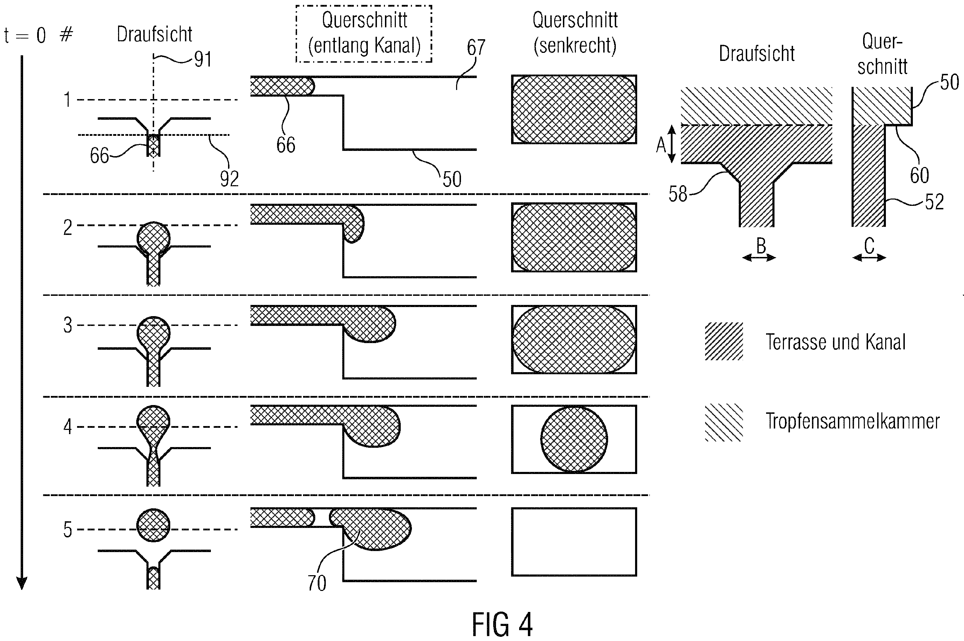

Fig. 4 zeigt fünf Phasen bei der Erzeugung eines Tropfens an der zweiten Aufweitung, unter Verwendung von Fluidikstrukturen, wie sie in Fig. 3b und bei I in Fig. 3c gezeigt sind. Entsprechende Fluidikstrukturen sind im rechten Bereich von Fig. 4 gezeigt. Der dort gezeigte Parameter A entspricht der Terrassenlänge (also der Länge des Bereichs gleichen Querschnitts), der Parameter B entspricht der Fluidkanalbreite und der Parameter C entspricht der Fluidkanaltiefe. Ferner sind in Fig. 4 jeweilige Querschnitte entlang des Kanals (entlang einer Linie q1) und jeweilige Querschnitte senkrecht zum Kanal (entlang einer Linie q2) gezeigt.

Zu Beginn des Verfahrens wird die Fluidikstruktur mit der zweiten Flüssigkeit 67, beispielsweise Öl, gefüllt. In Phase 1 wird die erste Flüssigkeit 66 zentrifugal durch den Fluidkanal 52 zugeführt. In Phase 2 erreicht die erste Flüssigkeit 66 die erste Aufweitung 58 und breitet sich in der Breitenrichtung aus. in der Phase 3 erreicht die erste Flüssigkeit 66 die zweite Aufweitung 50 und breitet sich auch in der Höhenrichtung aus. Diese Ausbreitung setzt sich in Phase 4 fort, bis in Phase 5 ein Tropfen 70 im zentrifugalen Feld abreißt.

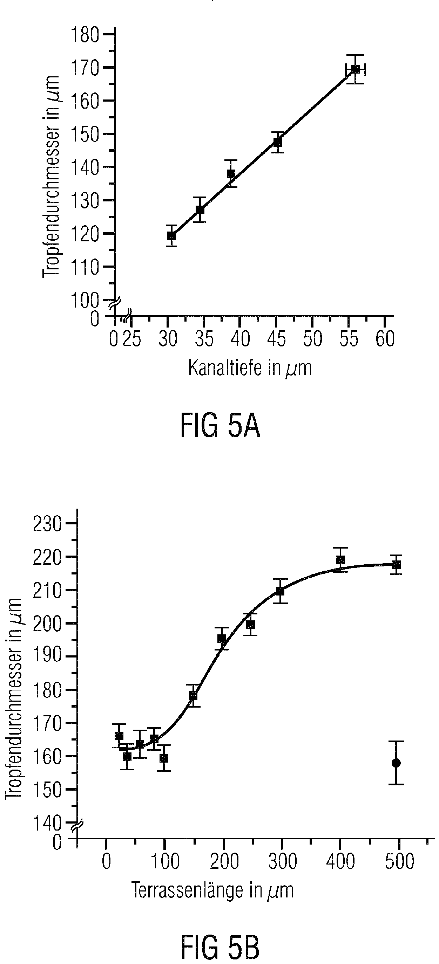

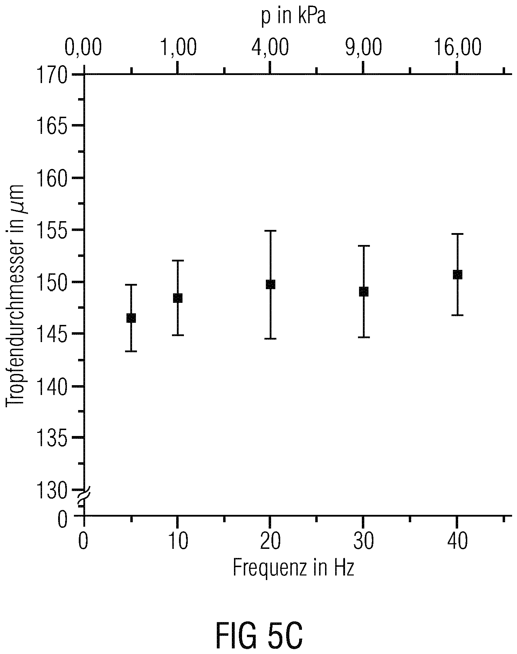

Die Figuren 5a bis 5c zeigen, wie sich unterschiedliche Parameter auf die Tropfengröße auswirken, wobei eine wässrige Phase als erste Flüssigkeit und Öl als zweite Flüssigkeit verwendet wurden. Fig. 5a zeigt wie sich eine Änderung der Fluidkanaltiefe C auf die Tropfengröße auswirkt. Fig. 5b zeigt, wie sich eine Änderung der Terrassenlänge A auf die Tropfengröße auswirkt. Fig. 5c zeigt, wie sich eine Änderung der Drehfrequenz auf die Tropfengröße auswirkt.

Die Erfinder haben herausgefunden, dass gute Maße für die Erzeugung mittelgroßer Tropfen wie folgt sind: A = 75 µm bis 125 µm, insbesondere 100 µm; B = 70 µm bis 110 µm, insbesondere 90 µm; und C = 45 µm bis 75 µm, insbesondere 60 µm. Der Winkel der ersten Aufweitung betrug 45°, kann aber geändert werden. Der Winkel der zweiten Aufweitung betrug 90°, kann aber auch abgesenkt werden. Die Tiefe der Fluidkammer (also der Tropfensammelkammer) betrug 200 µm.

Die Erfinder haben herausgefunden, dass die Aufweitung im ersten Aufweitungsbereich mindestens dem 1,1-fachen der Kanalbreite entsprechen sollte. Die Erfinder haben ferner herausgefunden, dass im zweiten Aufweitungsbereich ebenfalls mindestens eine 1,1-fache Aufweitung stattfinden sollte.

Wie in Fig. 5a zu erkennen ist, steigt die Tropfengröße linear mit der Kanaltiefe C. Die in Fig. 5a gezeigten Tropfengrößen wurden bei einer Terrassenlänge A von 100 µm, und einer Kanalbreite B von 90 µm erhalten.

Wie in Fig. 5b zu erkennen ist, sind bei einer Variation der Terrassenlänge A drei Teilbereiche der Kurve zu unterscheiden. Ist die Terrasse deutlich kürzer als die Kanalbreite so ist die Terrassenlänge für den Tropfendurchmesser irrelevant. Wird die Terrasse länger, steigt der Tropfendurchmesser mit der Terrassenlänge A (laut Theorie zur Potenz 2/3). Wird die Terrasse verglichen mit der Kanaltiefe C sehr lang, reißen die Tropfen schon auf der Terrasse ab und der Tropfendurchmesser bleibt ungefähr gleich. Teilweise entstehen Satellitentropfen, wie durch einen Tropfen mit einem Durchmesser von ca. 150 µm bei einer Terrassenlänge von 500 µm gezeigt ist. Die in Fig. 5b gezeigten Ergebnisse wurden bei einer konstanten Kanalbreite B von 90 µm und einer konstanten Kanaltiefe C von 60 µm erhalten.

Fig. 5c zeigt die Tropfengröße bei einer Variation des Drucks für eine konstante Struktur mit einer Kanalbreite B von 90 µm, einer Kanaltiefe C von 60 µm und einer Terrassenlänge A von 100 µm. Der Überdruck der wässrigen Phase gegenüber der Ölphase an der Mündung des Fluidkanals in die Fluidkammer wurde geändert, wobei diese Änderung durch eine Änderung der Drehfrequenz erreicht wurde. Wie in Fig. 5c zu sehen ist, hängt die Tropfengröße im gemessenen Bereich nicht von der Drehfrequenz und damit auch nicht vom Druck ab.

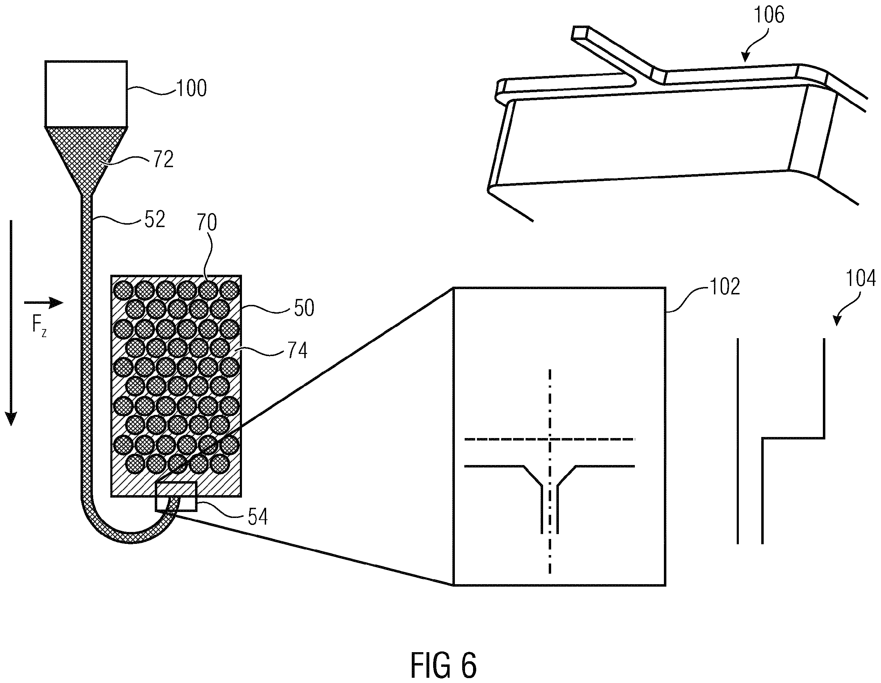

Fig. 6 zeigt schematisch ein Ausführungsbeispiel für eine Vorrichtung zur Durchführung eines entsprechenden Verfahrens, wobei die Vorrichtung in einem Zustand gezeigt ist, in dem eine Mehrzahl von Tropfen 70 einer ersten Flüssigkeit 72, die über einen Fluidkanal 52 zugeführt wird, in einer zweiten Flüssigkeit 74, die in einer Fluidkammer 50 angeordnet ist, erzeugt wurden. Ein Einlassbereich des Fluidkanals 52 kann mit einer Einlasskammer 100 fluidisch verbunden sein. Eine vergrößerte Draufsicht 102 und ein vergrößerter Längsschnitt 104 des Übergangsbereichs 54 sind in Fig. 6 ebenfalls dargestellt. Ein Fluidikmodul, das entsprechende Fluidikstrukturen aufweist, kann beispielsweise als eine Kartusche 106, wie in Fig. 6 schematisch dargestellt ist, in einen Rotor eingesetzt werden.

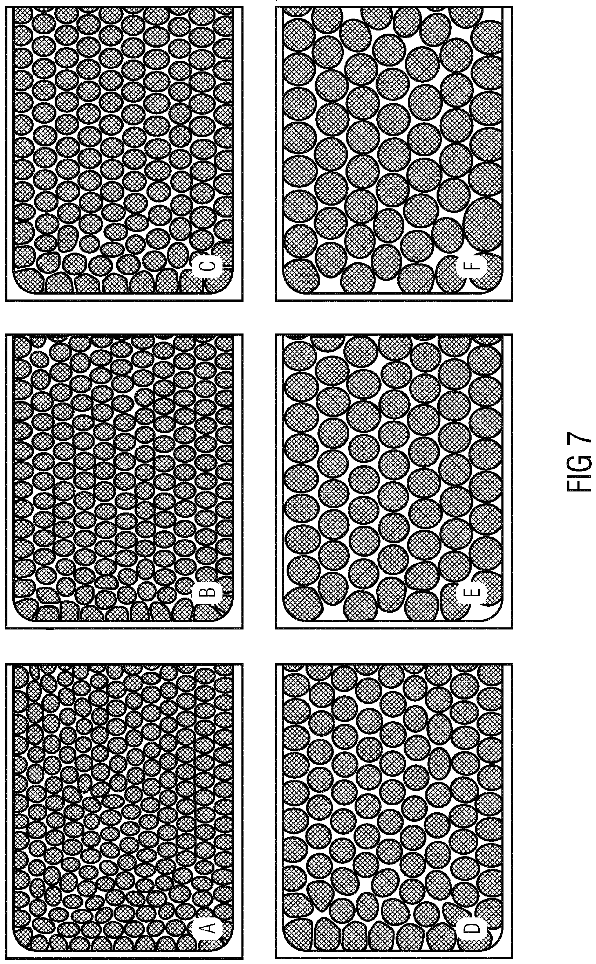

Ausführungsbeispiele der offenbarungsgemässen Fluidikstrukturen ermöglichen die zentrifugale mikrofluidische Erzeugung von Tropfen mit geringem manuellem Aufwand. In Fig. 7 sind in den Abschnitten A bis F jeweils Mikroskopaufnahmen (Draufsichten) von Fluidkammern gezeigt, in denen Tropfen verschiedener Größe erzeugt wurden. Diese Tropfen wurden mit verschieden dimensionierten Fluidikstrukturen erzeugt. Genauer gesagt wurden die Tropfen mit Fluidkanälen unterschiedlichen Querschnitts erzeugt, wobei für den Querschnitt (Größe) gilt: A<B<C<D<E<F. Alle Tropfen konnten mit nur zwei Pipettierschritten innerhalb von weniger als einer Minute bei unveränderter Drehfrequenz erzeugt werden, was einen deutlichen Vorteil gegenüber derzeit üblicherweise eingesetzten Systemen, die auf Spritzenpumpen basieren, darstellt.

Ausführungsbeispiele der Offenbarung basieren ferner auf der Erkenntnis, dass eine Tropfenerzeugung auf einer zentrifugalen mikrofluidischen Plattform leicht mit anderen Operationen auf derselben zentrifugalen mikrofluidischen Plattform kombiniert werden kann. Beispielsweise kann eine DNA-Extraktion mit anschließender DNA-Aufreinigung und anschließendem Mischen mit Komponenten zur Amplifikation von DNA und anschließendem Aliquotieren in viele kleine Tropfen und anschließender digitaler Amplifikation der DNA in den Aliquots mit minimalem Handhabungsaufwand und geringem Kontaminationsrisiko unter Verwendung der hierin beschriebenen Tropfenerzeugung realisiert werden.

Ausführungsbeispiele der Offenbarung eignen sich somit insbesondere auch für Verfahren im Zusammenhang mit biochemischen Nachweisreaktionen durch Aufteilung des Analyten in N Partitionen. Bei solchen Verfahren wird der Analyt soweit verdünnt, dass mindestens eine Partition und maximal N-1 Partitionen keinen Analyten enthalten. Durch Zählen der mit Analyt befüllten Partitionen kann die Konzentration des Analyten durch dessen Poisson-Verteilung zurückgerechnet werden. Das Aufteilen des Analyten (erste Flüssigkeit) kann dabei gemäß durch eine Tropfenerzeugung wie sie hierin beschrieben ist erfolgen.

Beispielsweise haben die Erfinder unter Verwendung der hierin beschriebenen Tropfenerzeugung erstmalig erfolgreich eine digitale Tropfen-RPA (RPA = Recombinase Polymerase Amplifikation) durchgeführt. Dabei wurden verdünnte kommerziell erhältliche DNA-Zielmoleküle mit kommerziell erhältlichem RPA-Mix versetzt und in Öl in viele verschiedene Tropfen (Reaktionsvolumina) unterteilt. Dies erfolgte wie bei den in Fig. 7 gezeigten Tropfen mit zwei Pipettierschritten und unveränderter Drehfrequenz. Es wurden keine Änderungen an der Zusammensetzung der kommerziellen Reagenzien vorgenommen. Anschließend wurden die Tropfen in der mikrofluidischen Struktur einer konstanten Temperatur ausgesetzt, um eine Enzymatische Reaktion (RPA) zu ermöglichen. Das Auslesen der Fluoreszenzintensität erfolgte ebenfalls in der mikrofluidischen Struktur mittels eines kommerziell erhältlichen Fluoreszenzscanners.

Bei weiteren Ausführungsbeispielen können die Fluidikstrukturen in einem Fluidikmodul auf einer Größe integriert sein, die im Wesentlichen denen eines Mikroskopobjektträgers (ca. 25x75 mm²) entsprechen. Durch Einlegen des Fluidikmoduls in eine Zentrifuge (z.B. eine Tischzentrifuge) können Tropfen in einem Bereich mit der Größe des Objektträgers erzeugt werden.

Bei weiteren Ausführungsbeispielen können die Fluidikstrukturen in einem Fluidikmodul auf einer Mikrotiterplatte, beispielsweise einer 96-Well-Platte, integriert sein. Durch Einlegen des Fluidikmoduls in eine Zentrifuge können Tropfen in einzelnen Vertiefungen der Platte erzeugt werden. Bei einem weiteren Ausführungsbeispiel sind die Fluidikstrukturen des Fluidikmoduls auf einem Einsatz für eine Mikrotiterplatte integriert. Durch Einlegen des Fluidikmoduls in eine Zentrifuge werden Tropfen in den einzelnen Vertiefungen der Platte erzeugt. Nach dem Erzeugen der Tropfen kann der Einsatz wieder aus der Mikrotiterplatte entfernt werden und die Tropfen für nachfolgende Anwendungen, wie beispielsweise eine PCR verwendet werden.

Bei Ausführungsbeispielen können die Tropfen somit eine biochemische Reaktionsmischung, die zur Detektion von DNA geeignet ist, enthalten, beispielsweise einen PCR-Mix oder verschiedene isothermale Amplifikationsmixe, wie z.B. RPA (Rekombinase Polymerase Amplifikation), RCA (Rolling Circle Amplifikation), LAMP (Loop-mediated Isothermal Amplifikation) oder verschiedene Mixe zur nicht-isothermalen DNA-Detektion. Überdies können manche Tropfen DNA-Moleküle, die nachgewiesen werden sollen, enthalten. Das komplette Fluidikmodul, in dem die Fluidikstrukturen gebildet sind, kann eine standardisierte Größe aufweisen. Das gesamte Fluidikmodul kann nach Erzeugung der Tropfen z.B. mit konventionellen Geräten, sogenannten Slidecyclern, verschiedenen Temperaturen ausgesetzt werden, um eine DNA-Nachweisreaktion (z.B. PCR, RPA, RCA, LAMP) zu ermöglichen. Diese Nachweisreaktion kann z.B. über einen fluoreszierenden Farbstoff nachgewiesen werden, der nach oder während der Reaktion über ein optisches System ausgelesen werden kann. Dies kann auf Grund der standardisierten Größe z.B. in einem sogenannten Slide-Scanner durchgeführt werden. Hierzu können Teile des Systems transparent ausgestaltet sein. Außerdem besteht die Möglichkeit, die Temperatur des Fluidikmoduls während des kompletten Prozesses zu kontrollieren, z.B. um eine vorzeitige Aktivierung von Enzymen durch niedrige Temperaturen zu vermeiden. Unter isothermalen Amplifikationsverfahren sind dabei Amplifikationsverfahren zu verstehen, die bei einer konstanten Temperatur stattfinden.

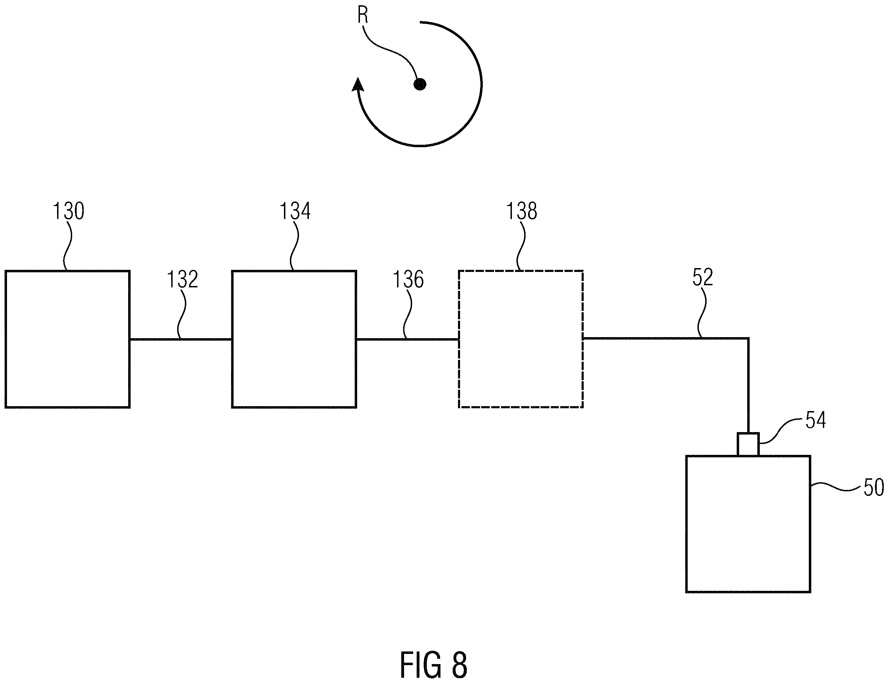

Ausführungsbeispiele schaffen eine Vorrichtung, bei der die Tropfengenerierungsstruktur auf einer zentrifugalen mikrofluidischen Plattform mit einer zentrifugalen mikrofluidischen Struktur verbunden ist, die eine DNA-Extraktion und/oder Aufreinigung erlaubt, bevor die DNA mit einer biochemischen Reaktionsmischung versetzt wird, die einen Nachweis der DNA auf Basis von z.B. PCR oder anderen z.B. isothermalen oder nicht isothermalen Amplifikationsverfahren ermöglicht. Zum Beispiel kann eine digitale PCR oder digitale RPA nachgeschaltet werden. Ein Beispiel für entsprechende Fluidikstrukturen ist in Fig. 8 gezeigt. Auf einer zentrifugalen mikrofluidischen Plattform ist ein Einlass 130 über einen Kanal 132 mit Strukturen 134 für eine DNA-Extraktion und/oder -Aufreinigung 134 verbunden. Strömungsmäßig davor oder danach kann das System über einen Kanal 136 mit weiteren Strukturen 138 für automatisierte Manipulationsschritte (z.B. Voramplifikation oder eine doppelte Tropfenerzeugung z.B. mit zwischengeschalteter Voramplifikation) verbunden sein. Diese Strukturen 138 oder die Strukturen für die DNA-Extraktion und/oder -Aufreinigung 134 sind über einen Fluidkanal 52 mit einem Übergang 54 zur Tropfenerzeugung und einer Fluidkammer 50 verbunden. Somit kann die Tropfenerzeugung mit anderen fluidischen Operationen über Kanäle sowie mit einem Einlass verbunden sein, was beispielsweise eine automatisierte DNA-Extraktion, DNA-Aufreinigung, DNA-Voramplifikation und anschließend eine digitale PCR (bzw. digital isothermale Nachweisverfahren) ermöglicht.

Bei alternativen Ausführungsbeispielen können statt DNA auch andere Nukleinsäuren nachgewiesen und detektiert werden, wie z.B. RNA (Ribonukleinsäure).

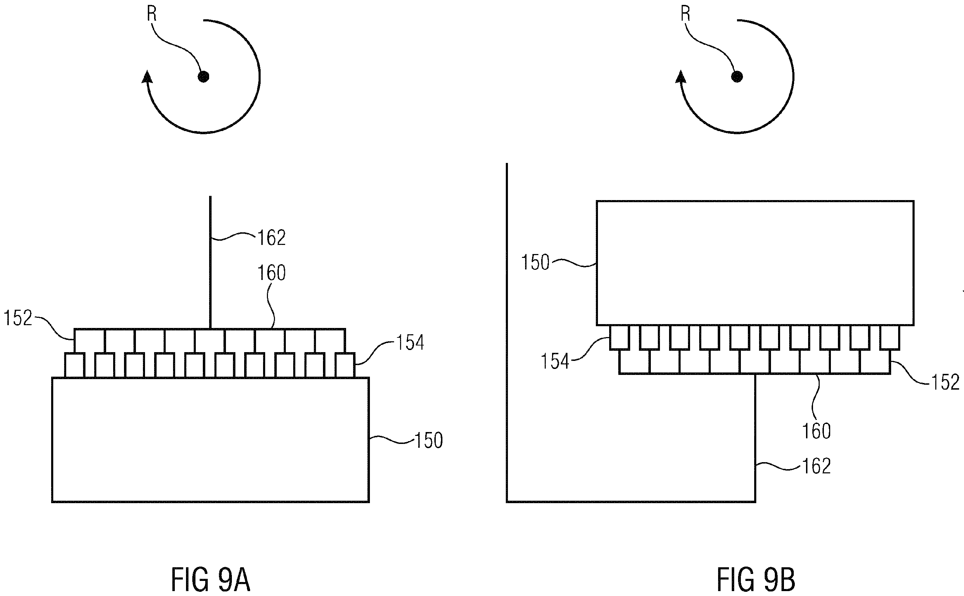

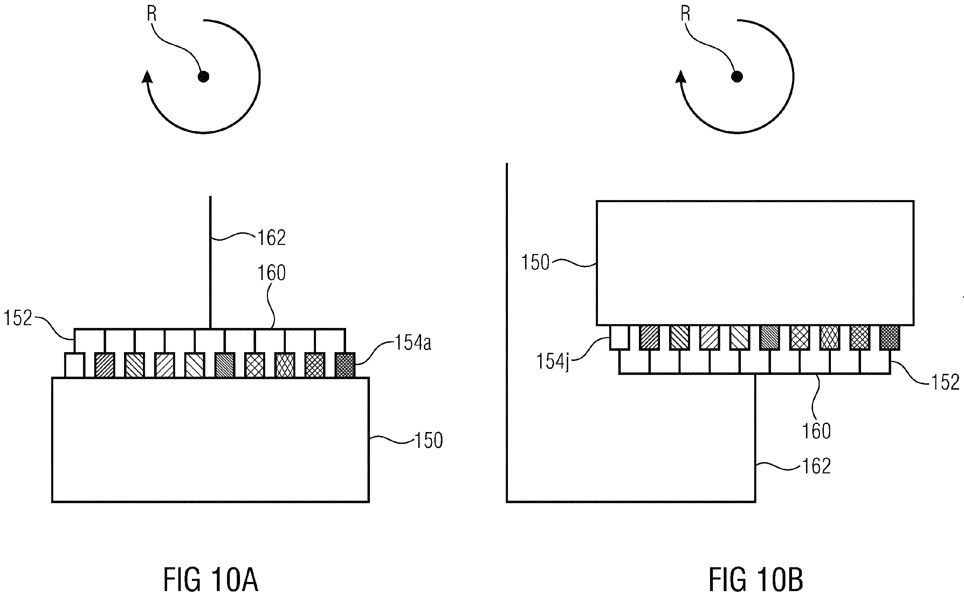

Bezugnehmend auf die Figuren 9 und 10 werden nachfolgend Ausführungsbeispiele erläutert, durch die die Tropfenproduktionsrate gesteigert werden kann, indem mehrere Erzeugungsstrukturen parallel geschaltet werden, so dass gleichzeitig mehrere Tropfen erzeugt werden können.

Fig. 9a zeigt Fluidikstrukturen, bei denen jeder einer Mehrzahl von Fluidkanälen 152 in einem entsprechenden Übergangsbereich 154 in eine Fluidkammer 154 mündet. Die Fluidkanäle 152 sind über eine Verteilerstruktur 160 mit einem gemeinsamen Zuführkanal 162 fluidisch verbunden. Statt eines Zuführkanals könnten auch mehrere Zuführkanäle vorgesehen sein, wobei eine erste Teilmenge der Fluidkanäle über einen ersten Verteilerkanal mit einem ersten mit einem ersten Zuführkanal und eine zweite Teilmenge der Fluidkanäle über einen zweiten Verteilerkanal mit einem zweiten Zuführkanal verbunden sein könnte. Die Fluidkanäle münden in einem radial inneren Abschnitt der Fluidkammer in dieselbe, so dass die Fluidikstrukturen für eine erste Flüssigkeit geeignet sind, die eine größere Dichte aufweist als die zweite Flüssigkeit. Die Übergangsbereiche 154 weisen einen identischen Aufbau auf, so dass Tropfen gleicher Größe parallel erzeugt werden können.

Fig. 9b zeigt ähnliche Fluidikstrukturen, wobei die Fluidkanäle jedoch in einem radial äußeren Abschnitt derselben in die Fluidkammer münden, so dass die Fluidikstrukturen für eine erste Flüssigkeit geeignet sind, die eine geringere Dichte aufweist als die zweite Flüssigkeit.

Somit schaffen Ausführungsbeispiele ein Fluidikmodul, das um ein Rotationszentrum R drehbar ist, bei dem ein Zuführkanal 162 mit einem Verteilerkanal 160 gekoppelt ist, von dem mehrere Fluidkanäle 152 abzweigen, welche durch mehrere Übergänge 154 in eine Fluidkammer 150 überführt werden. Die Übergänge 154 zwischen der Fluidkammer 150 und den Fluidkanälen 152 sind wiederum so gestaltet, dass eine Strömung einer ersten Flüssigkeit, die in der zweiten Flüssigkeit nicht mischbar ist, durch die Kanäle in Richtung der Fluidkammer, hervorgerufen durch Rotation des Fluidikmoduls und einen dadurch entstehenden hydrostatischen Druck, das Entstehen von Tropfen der ersten Flüssigkeit eingebettet in der zweiten Flüssigkeit hervorruft. Hierbei fließt nur die erste Phase signifikant.

Die Figuren 10a und 10b zeigen Fluidikstrukturen, die ähnlich zu denen in den Figuren 9a und 9b sind. Jedoch sind in den Figuren 10a und 10b die Übergänge unterschiedlich dimensioniert, so dass unterschiedlich große Tropfen entstehen. Dies ist in den Figuren 10a und 10b angedeutet, indem die Übergänge unterschiedlich schraffiert sind, wobei lediglich beispielhaft zwei Übergänge mit den Bezugszeichen 154a und 154j bezeichnet sind. Bei weiteren Ausführungsbeispielen können somit verschieden große Tropfen parallel produziert werden. Ausführungsbeispiele der vorliegenden Offenbarung schaffen somit ein Fluidikmodul, das um ein Rotationszentrum R drehbar ist, bei dem ein Zuführkanal 162 mit einem Verteilerkanal 160, von dem mehrere Fluidkanäle 152 abzweigen, gekoppelt ist. Die Fluidkanäle 152 werden durch mehrere strukturell ähnliche aber in ihren Ausmaßen unterschiedlich gestaltete Übergänge 154a, 154j in eine Fluidkammer 150 überführt. Die Übergänge 154a, 154j zwischen der Fluidkammer und dem Fluidkanal sind so gestaltet, dass eine Strömung einer ersten Flüssigkeit, die in der zweiten Flüssigkeit nicht mischbar ist, durch die Fluidkanäle in Richtung der Fluidkammer, hervorgerufen durch Rotation des Fluidikmoduls und einen dadurch entstehenden hydrostatischen Druck, das Entstehen von Tropfen unterschiedlicher Größe der ersten Flüssigkeit eingebettet in der zweiten Flüssigkeit hervorruft. Hierbei fließt nur die erste Phase signifikant. Somit können Tropfen mit unterschiedlichen, aber definierten Größen beispielsweise in einer Kartusche mit einer Rotationsfrequenz erzeugt werden.

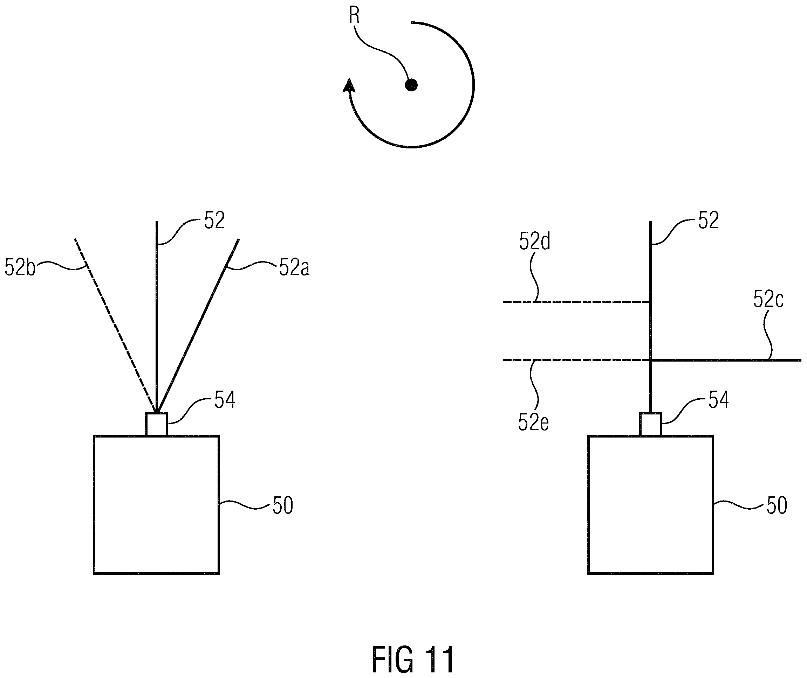

Generell wird bei Ausführungsbeispielen der Offenbarung zur Erzeugung eines Tropfens nur ein Fluidkanal benötigt, der in einem Übergangsbereich in eine Fluidkammer mündet. Bei weiteren Ausführungsbeispielen können ein oder mehrere Kanäle Flüssigkeiten kurz vor dem oder im Übergang zusammenführen. Dies ermöglicht die Mischung von Substanzen unmittelbar vor der Erzeugung von Tropfen. Dies ermöglicht außerdem das gezielte Erzeugen von anisotropen Tropfen z.B. die Herstellung von Januspartikeln oder ähnlichem. Mögliche Fluidikstrukturen zum Zusammenführen von mindestens zwei Kanälen vor der Tropfenerzeugung sind in Fig. 11 gezeigt. Bei den linken Fluidikstrukturen laufen zwei Kanäle 52 und 52a in einen gemeinsamen Übergang 54, der Tropfen erzeugt, die sich in eine Kammer 50 bewegen. Ein oder mehrere weitere Kanäle, die mit den Kanälen 52 und 52a zusammenlaufen, können vorgesehen sein, wie durch einen optionalen Kanal 52b in gestrichelten Linien angedeutet ist. Bei den rechten Fluidikstrukturen mündet ein weiterer Kanal 52c vor einem Übergang 54, der Tropfen erzeugt, in einen Hauptkanal 52. Ein oder mehrere weitere Kanäle 52d, 52e können ebenfalls in den Hauptkanal 52 münden, wie in gestrichelten Linien angedeutet ist. Dabei können die mündenden Kanäle 52c, 52d, 52e am gleichen Ort und/oder an unterschiedlichen Orten in den Hauptkanal 52 münden.

Bei weiteren Ausführungsbeispielen können Tropfen einer ersten Phase produziert werden, die in einer zweiten Phase eingebettet sind und in ihrem Inneren eine dritte mit der ersten Phase nicht mischbare Phase enthalten. Dies können beispielsweise Tropfen einer Suspension, z.B. Zellen oder Beads, sein. Außerdem können es z.B. Tropfen einer Emulsion sein. Mindestens eine der Phasen kann in einem späteren Schritt (teilweise) gehärtet werden. Dadurch wird unter anderem die Herstellung von Januspartikeln, z.B. für die Verkapselung von Medizinprodukten, ermöglicht.

Bei weiteren Ausführungsbeispielen können Tropfen produziert werden, von denen manche Bakterien enthalten. Diese Bakterien können über eine Nachweisreaktion (z.B. fluoreszente Phagen) nachgewiesen werden und über eine geeignete Detektionsmethode (z.B. Fluoreszenzmessung) detektiert werden. Dies ermöglicht eine absolute Quantifizierung von viablen Bakterien, zum Beispiel zur Diagnose von Sepsis.

Bei weiteren Ausführungsbeispielen können Tropfen Bestandteile für die Durchführung einer Immunonachweisreaktion (Immunoassay) enthalten, die es ermöglicht, Antigene oder Antikörper nachzuweisen. Wird die Anzahl der Tropfen so angepasst, dass weder alle noch keine Tropfen die entsprechenden Antigene oder Antikörper enthalten kann so eine digitaler Nachweis von Antigenen oder Antikörper (z.B. Digitale ELISA (Enzyme Linked Immunosorbent Assay)) durchgeführt werden, was unter anderem eine absolute Quantifizierung von Antigenen oder Antikörpern ermöglicht.

Die Erfinder haben erkannt, dass die bekannten Systeme zur Tropfenerzeugung, wie sie eingangs beschrieben sind, an zahlreichen Nachteilen leiden.

So wird bei druckgetriebenen mikrofluidischen Systemen zum Aliquotieren für die Erzeugung von Tropfen ein externes System zum Aufbau eines geeigneten Drucks benötigt. Dies zieht eine Reihe von Nachteilen mit sich. So werden Geräte zur Erzeugung von Druck für den Betrieb der druckgetriebenen mikrofluidischen Systeme benötigt, die keine Standardgeräte sind, sodass für jede Anwendung kostspielige spezialisierte Systeme erforderlich sind. Je nach Ausführung ist der Betrieb dieser Systeme komplex, da zum Beispiel eine dichte Verbindung zwischen dem System und der Kartusche zur Erzeugung der Tropfen sichergestellt werden muss. Technische Lösungen für dieses Problem sind denkbar, erhöhen aber Spezialisierungsgrad und Kosten. Druckschwankungen in den Systemen zum Druckaufbau können nur unter hohen Kosten minimiert werden und führen zu Schwierigkeiten beim Betrieb der Systeme. Die Grundoperation des Aliquotierens in druckgetriebenen Systemen lässt sich nur mit großem Aufwand mit anderen Grundoperationen kombinieren. Ein denkbares monolithisches System das z.B. die DNA-Extraktion, DNA-Aufreinigung und eine digitale PCR automatisiert, ließe sich druckgetrieben nur unter großen Schwierigkeiten realisieren oder würde sehr kompliziert zu bedienen sein, was die Anwendung in Standardsituationen stark erschwert. Insgesamt sind druckgetriebene Systeme teuer, unterliegen Druckschwankungen durch Pulsieren und sind durch eine komplexe Integration gekennzeichnet.

Auch bisherige zentrifugale mikrofluidische Systeme zum Aliquotieren weisen zahlreiche Nachteile auf. So ist das Erzeugen von Tropfen, die durch Umgebungsluft in ein Auffanggefäß fliegen, durch eine Reihe von Nachteilen beschränkt. Das System ist nur begrenzt auf andere Flüssigkeiten anwendbar, da das (teilweise) Aushärten der Tropfen für den Betrieb des Systems unerlässlich ist. Eine Kontamination der Umgebung und/oder der Tropfen lässt sich während des Kontakts zur Umgebungsluft nicht ausschließen. Die Grundoperation des Aliquotierens in diesen Systemen lässt sich nur mit großem Aufwand mit anderen Grundoperationen kombinieren. Ein denkbares monolithisches System, das z.B. die DNA-Extraktion, DNA-Aufreinigung und eine digitale PCR automatisiert, ließe sich nur unter großen Schwierigkeiten realisieren oder würde sehr kompliziert zu bedienen sein, was die Anwendung in Standardsituationen stark erschwert. Ferner ist es unter Verwendung solcher Verfahren sehr schwer, besonders kleine Tropfen herzustellen.

Bei mikrovertiefungsbasierten mikrofluidischen Systemen zum Aliquotieren ist das Erzeugen von Aliquots in Vertiefungen auf einer sich drehenden Scheibe durch eine Reihe von Nachteilen beschränkt. Es bestehen große Schwierigkeiten bei der nachfolgenden Verarbeitung (Downstreamverarbeitung) der Aliquots, die z.B. zur Erzeugung von Januspartikein für Medizinprodukte unerlässlich ist. Der Platzverbrauch der Vertiefungen ist relativ hoch, da sie nicht 3-dimensional angeordnet werden können und die starren Wände zwischen den Vertiefungen eine gewisse Breite haben, die größer ist als der Abstand zwischen eng gepackten Tropfen.

Die Erfinder haben ferner erkannt, dass bei dem in [4] und [21] beschriebenen Verfahren das zugrundeliegende physikalische Prinzip des Tropfenabrisses stark von den Flussraten des Öls und der zu emulgierenden Phase abhängig ist. Da zu Beginn und zum Ende des Prozesses die Flussraten nicht genau kontrolliert werden können, führt dies zu inhomogenen Tropfen am Anfang und Ende des Prozesses. Da ferner zur Erzeugung der Tropfen ein ständiger Fluss der Ölphase notwendig ist, wird zur Erzeugung der Emulsion eine große Menge des Öls benötigt. Ferner benötigen solche bisherige Systeme mindestens drei Kanäle zur Erzeugung der Tropfen, was zu einem erhöhten Platzverbrauch auf der Scheibe führt, verglichen mit nur einem Kanal, wie er bei Ausführungsbeispielen der Offenbarung benötigt wird. Darüber hinaus erfordert die Anpassung auf andere Tropfenvolumina bei einem solchen Ansatz ein komplettes Neudesign der Strukturen. Im hier vorgestellten System muss im Wesentlichen nur der Durchmesser eines einzigen Kanals angepasst werden.

Die bezüglich des Stands der Technik genannten Nachteile können durch den offenbarungsgemässen Lösungsansatz zu großen Teilen oder vollständig ausgeräumt werden. Erfindungsgemäß können zentrifugal erzeugte Antriebskräfte für einen Tropfenabriss genutzt werden. Der Dichteunterschied zwischen zwei nicht mischbaren Flüssigkeiten kann genutzt werden, um eine Emulsion zu erzeugen. Weiterhin ist der erfindungsgemäße Lösungsansatz vorteilhaft dahingehend, dass das zentrifugale Feld, das zur Tropfenerzeugung verwendet wird, gleichzeitig ausgenutzt werden kann, um die erzeugten Tropfen vom Ort der Erzeugung weg zu bewegen und die umgebende Phase am Ort der Tropfenerzeugung zu halten. Beispielsweise können Tropfen durch zentrifugal erzeugte Auftriebskräfte von der Mündung der Fluidkanals in die Fluidkammer weggeführt werden. Künstliche Auftriebskräfte können verwendet werden, um die zweite Flüssigkeit an der Mündung des Fluidkanals in die Fluidkammer zu halten, wodurch hohe Wasser/Öl-Verhältnisse in der Emulsion realisiert werden können. Ausführungsbeispiele benötigen nur einen Kanal, der in eine Kammer führt, für eine entsprechende Tropfenerzeugung.

Ferner werden im Gegensatz zu den momentan am meisten verwendeten Methoden zu Erzeugung von Tropfen erfindungsgemäß keine externen Druckquellen benötigt, was die Fehleranfälligkeit und die Kosten senkt. Die vorliegende Erfindung kann im Gegensatz zum Stand der Technik mit Standardlaborgeräten (z.B. Tischzentrifugen) betrieben werden. Weiterhin ist es leicht möglich, entsprechende Geräte für spezielle Anwendungsgebiete zu entwickeln und zu bauen. Durch den erfindungsgemäßen Lösungsansatz kann die Handhabung gegenüber dem Stand der Technik deutlich vereinfacht werden, da beispielsweise nur ein Pipettierschritt und eine Operation in einem Standardlaborgerät nötig ist. Das Kontaminationsrisiko kann gesenkt werden. Ausführungsbeispiele können mit nur einem bis wenigen Pipettierschritten beladen werden. Eine folgende Aliquotierung lässt sich unter Verwendung des erfindungsgemäßen Lösungsansatzes leicht automatisieren. Der erfindungsgemäße Ansatz vereinfacht gegenüber dem Stand der Technik das Kombinieren von Aliquotieren und anderen Prozessschritten, wie z.B. der Extraktion und Aufreinigung von DNA. Ferner kann die Menge an eingesetzter umgebender Phase im Gegensatz zum Stand der Technik deutlich verringert werden. Im Gegensatz zum Stand der Technik ist der erfindungsgemäße Ansatz in der Lage, totvolumenfrei das komplette Probenvolumen zu aliquotieren bzw. zu emulgieren. Ferner ermöglicht er weitgehende nachfolgende Manipulationen (Downstreammanipulationen) des aliquotierten Probenvolumens. Außerdem können die erzeugten Tropfen durch schnelle Zentrifugation wieder aufgebrochen und zu einem Gesamtvolumen zusammengeführt werden. Dies ist für manche Anwendungen, z.B. eine Sequenzierung oder eine Voramplifikation, notwendig und in druckgetriebenen Systemen nur sehr umständlich, beispielweise durch Zugabe von Chemikalien, umsetzbar.

Der erfindungsgemäße Lösungsansatz nutzt ein weitestgehend passives System, dessen einziger Freiheitsgrad (Drehfrequenz) genutzt werden kann, um viele weitere vorgelagerte Prozesse zu steuern. Im Gegensatz zu druckbetriebenen Systemen existieren keine Startprobleme, d.h. es können von Beginn an bis zum Schluss homogene Tropfen erzeugt werden. Ferner ermöglicht der erfindungsgemäße Ansatz ein einfaches Einstellen der Flussraten über das Frequenzprotokoll.

Ausführungsbeispiele der Offenbarung ermöglichen die zentrifugale Erzeugung von Flüssigin-Flüssig-Tropfen aus zwei flüssigen Phasen, wobei im Wesentlichen nur eine Phase fließt. Ausführungsbeispiele benötigen nur einen Fluidkanal, der in die Fluidkammer führt, zur Erzeugung jeweils eines Tropfens, wobei mehrere Kanäle vorgesehen sein können, um parallel mehrere Tropfen zu erzeugen. Ausführungsbeispiele ermöglichen einen digitalen Nachweis von Zielmolekülen (z.B. einer DNA-Amplifikation) auf dem Rotationskörper. Ausführungsbeispiele schaffen ein Substrat, das eine Kammer, die mit einem Fluidkanal verbunden ist, aufweist, wobei der Übergang von Fluidkanal zu Kammer so ausgestaltet ist, dass nach einer Befüllung der Kammer mit einer zweiten flüssigen Phase (z.B. Öl) die Strömung einer ersten, in der ersten nicht mischbaren, flüssigen Phase (z.B. Wasser), hervorgerufen durch Rotation des Substrats (aufgrund hydrostatischem Zentrifugaldruck) Tropfen generiert werden (wobei im Wesentlichen nur eine der beiden Phasen während der Tropfenbildung fließt). Der Durchmesser der erzeugten Tropfen kann größer sein als die kleinste Kanaldimension des Übergangs.

Ausführungsbeispiele der Offenbarung schaffen somit eine mikrofluidische Struktur zur Generierung von Tropfen innerhalb einer mikrofluidischen Kartusche, welche zentrifugal betrieben wird. Bei einer entsprechenden Erzeugung von Tropfen fließt maßgeblich nur die zu vereinzelnde Flüssigkeit. Tropfenabriss und Tropfenvolumen sind hauptsächlich durch Kapillarkräfte, Auftriebskräfte, Oberflächenspannung und Geometrie des Übergangbereichs (Düsengeometrie) bestimmt. Das Volumen der erzeugten Tropfen ist in großem Bereich unabhängig von Flussrate und Druck.

Obwohl einige Aspekte hierein im Zusammenhang mit einer Vorrichtung und der Funktionalität einer Vorrichtung beschrieben sind, ist klar, dass diese Aspekte auch eine Beschreibung eines entsprechenden Verfahrens liefern. In gleicher Weise ist klar, dass einige Aspekte, die im Zusammenhang mit einem Verfahren beschrieben wurden, auch eine Beschreibung einer Vorrichtung betreffen, die entsprechend ausgelegt ist, um eine dem Verfahren entsprechende Funktionalität zu liefern.

### Literaturverzeichnis

[1] Dangla, Remi; Fradet, Etienne; Lopez, Yonatan; Baroud, Charles N. (2013): The physical mechanisms of step emulsification, in: Journal of Physics D: Applied Physics 46 (11), S. 114003
[2] Dangla, Remi; Kayi, S. Cagri; Baroud, Charles N. (2013): Droplet microfluidics driven by gradients of confinement, in: Proc. Natl. Acad. Sci. U.S.A. 110 (3), S. 853-858. DOI: 10.1073/pnas.1209186110
[3] Haeberle, Stefan; Naegele, Lars; Burger, Robert; Stetten, Felix von; Zengerle, Roland; Ducree, Jens (2008): Alginate bead fabrication and encapsulation of living cells under centrifugally induced artificial gravity conditions, in: J Microencapsul 25 (4), S. 267-274. DOI: 10.1080/02652040801954333
[4] Haeberle, Stefan; Zengerle, Roland; Ducree, Jens (2007): Centrifugal generation and manipulation of droplet emulsions, in: Microfluidics and Nanofluidics 3 (1), S. 65-75
[5] Kan, Cheuk W.; Rivnak, Andrew J.; Campbell, Todd G.; Piech, Tomasz; Rissin, David M.; Mösl, Matthias et al. (2012): Isolation and detection of single molecules on paramagnetic beads using sequential fluid flows in microfabricated polymer array assemblies, in: Lab Chip 12 (5), S. 977-985. DOI: 10.1039/c2lc20744c
[6] Kawakatsu, Takahiro; Kikuchi, Yuji; Nakajima, Mitsutoshi (1997): Regular-sized cell creation in microchannel emulsification by visual microprocessing method, in: Journal of the American Oil Chemists' Society 74 (3), S. 317-321
[7] Mark, Daniel; Haeberle, Stefan; Zengerle, Roland; Ducree, Jens; Vladisavljević, Goran T. (2009): Manufacture of chitosan microbeads using centrifugally driven flow of gel-forming solutions through a polymeric micronozzle, in: Journal of colloid and interface science 336 (2), S. 634-641
[8] Metz, Tobias; Paust, Nils; Zengerle, Roland; Koltay, Peter (2010): Capillary driven movement of gas bubbles in tapered structures, in: Microfluidics and Nanofluidics 9 (2-3), S. 341-355
[9] Sugiura, Shinji; Nakajima, Mitsutoshi; Iwamoto, Satoshi; Seki, Minoru (2001): Interfacial tension driven monodispersed droplet formation from microfabricated channel array, in: Langmuir 17 (18), S. 5562-5566
[10] Sugiura, Shinji; Nakajima, Mitsutoshi; Kumazawa, Naoyuki; Iwamoto, Satoshi; Seki, Minoru (2002a): Characterization of spontaneous transformation-based droplet formation during microchannel emulsification, in: The Journal of Physical Chemistry B 106 (36), S. 9405-9409
[11] Sugiura, Shinji; Nakajima, Mitsutoshi; Oda, Tatsuya; Satake, Mitsuo; Seki, Minoru (2004): Effect of interfacial tension on the dynamic behavior of droplet formation during microchannel emulsification, in: Journal of colloid and interface science 269 (1), S. 178-185
[12] Sugiura, Shinji; Nakajima, Mitsutoshi; Seki, Minoru (2002): Effect of Channel Structure on Microchannel Emulsification, in: Langmuir 18 (15), S. 5708-5712. DOI: 10.1021/la025813a
[13] Sugiura, Shinji; Nakajima, Mitsutoshi; Seki, Minoru (2002): Prediction of droplet diameter for microchannel emulsification, in: Langmuir 18 (10), S. 3854-3859
[14] Sugiura, Shinji; Nakajima, Mitsutoshi; Seki, Minoru (2002): Preparation of monodispersed polymeric microspheres over 50 µm employing microchannel emulsification, in: Industrial & engineering chemistry research 41 (16), S. 4043-4047
[15] Sugiura, Shinji; Nakajima, Mitsutoshi; Seki, Minoru (2004): Prediction of droplet diameter for microchannel emulsification: prediction model for complicated microchannel geometries, in: Industrial & engineering chemistry research 43 (26), S. 8233-8238
[16] Sugiura, Shinji; Nakajima, Mitsutoshi; Tong, Jihong; Nabetani, Hiroshi; Seki, Minoru (2000): Preparation of monodispersed solid lipid microspheres using a microchannel emulsification technique, in: Journal of colloid and interface science 227 (1), S. 95-103
[17] Sugiura, Shinji; Nakajima, Mitsutoshi; Ushijima, Hideki; Yamamoto, Koji; Seki, Minoru (2001b): Preparation Characteristics of Monodispersed Water-in-Oil Emulsions Using Microchannel Emulsification, in: Journal of chemical engineering of Japan 34 (6), S. 757-765
[18] Sugiura, Shinji; Nakajima, Mitsutoshi; Yamamoto, Koji; Iwamoto, Satoshi; Oda, Tatsuya; Satake, Mitsuo; Seki, Minoru (2004): Preparation characteristics of water-in-oilin-water multiple emulsions using microchannel emulsification, in: Journal of colloid and interface science 270 (1), S. 221-228
[19] Sugiura, Shinji; Nakajima, Mitsutoshi; Seki, Minoru (2002): Preparation of monodispersed emulsion with large droplets using microchannel emulsification, in: JOACS, Vol. 79, No. 5
[20] Sugiura, Shinji; Nakajima, Mitsutoshi; Itou, Hitsatsugu; Seki, Minoru (2001): Synthesis of polymeric microspheres with narrow size distributions employing microchannel emulsification, in: Macromol. Rapid Commun. 2001, 22, No. 10, Seiten 773 - 778, Wiley-VCH Verlag GmbH, D-69451 Weinheim.
[21] DE 10 2005 048 259 A1
[22] US 6 387 301 B1
[23] US 2013/0078164 A1

## Patentansprüche

1. Verfahren zum Erzeugen von einem oder mehreren Tropfen einer ersten Flüssigkeit in einer mit der ersten Flüssigkeit nicht mischbaren zweiten Flüssigkeit unter Verwendung einer Vorrichtung zur Erzeugung von einem oder mehreren Tropfen einer ersten Flüssigkeit in einer mit der ersten Flüssigkeit nicht mischbaren zweiten Flüssigkeit, wobei die Vorrichtung folgende Merkmale aufweist:
einen Rotationskörper (10), der Fluidikstrukturen aufweist, wobei die Fluidikstrukturen folgende Merkmale aufweisen:
eine Fluidkammer (50, 150), die ausgebildet ist, um die zweite Flüssigkeit zu enthalten;
einen Fluidkanal (52, 152), der in die Fluidkammer (50, 150) mündet und ausgebildet ist, um eine Fluss der ersten Flüssigkeit in einer Strömungsrichtung zu der Fluidkammer (50, 150) zu bewirken; und
einen Übergangsbereich (54, 154, 154a, 154j), in dem der Fluidkanal (52, 152) in die Fluidkammer (50, 150) mündet, wobei der Übergangsbereich (54, 154, 154a, 154j) einen ersten Aufweitungsbereich (54a) aufweist, in dem sich der Strömungsquerschnitt für den Fluss der ersten Flüssigkeit in zumindest einer ersten Richtung senkrecht zur Strömungsrichtung aufweitet, und einen zweiten Aufweitungsbereich (54b) aufweist, in dem sich der Strömungsquerschnitt für den Fluss der ersten Flüssigkeit in einer zweiten Richtung, die senkrecht zu der Strömungsrichtung und zu der ersten Richtung ist, aufweitet, wobei der zweite Aufweitungsbereich (54b) stromabwärts von dem ersten Aufweitungsbereich (54a) angeordnet ist; und
eine Antriebsvorrichtung (22), die ausgebildet ist, um den Rotationskörper (10) mit einer solchen Rotation zu beaufschlagen, dass die erste Flüssigkeit zentrifugal zu der Fluidkammer (50, 150) zugeführt wird und dass aufgrund des zweiten Aufweitungsbereichs (54b) zentrifugal-hydrodynamisch induzierte Druck-, Auftriebs- und Kapillar-Kräfte bewirkt werden, die einen Tropfenabriss in der ersten Flüssigkeit bewirken, so dass ein in der zweiten Flüssigkeit eingebetteter Tropfen der ersten Flüssigkeit erzeugt wird,
wobei die Vorrichtung zum Erzeugen eines Tropfens einer ersten Flüssigkeit mit einer ersten Dichte in einer zweiten Flüssigkeit mit einer zweiten Dichte ausgelegt ist, wobei a) die erste Dichte größer ist als die zweite Dichte und der Fluidkanal (52, 152) in einem radial inneren Bereich in die Fluidkammer (50, 150) mündet, oder b) die zweite Dichte größer ist als die erste Dichte und der Fluidkanal (52, 152) in einem radial äußeren Bereich in die Fluidkammer (50, 150) mündet,
wobei das Verfahren folgende Merkmale aufweist:
Drehen des Rotationskörpers (10), um die erste Flüssigkeit zentrifugal durch den Fluidkanal (52, 152) zu der Fluidkammer (50, 150), in die die zweite Flüssigkeit eingebracht ist, zuzuführen und um in dem zweiten Aufweitungsbereich die auf die erste Flüssigkeit wirkende, zentrifugal erzeugte Druckkraft, Auftriebskraft und Kapillarkraft so zu kontrollieren, dass ein Tropfenabriss der ersten Flüssigkeit bewirkt wird, so dass ein in der zweiten Flüssigkeit eingebetteter Tropfen der ersten Flüssigkeit erzeugt wird,
wobei nach dem Erzeugen des Tropfens der Tropfen aufgrund unterschiedlicher Dichten der ersten Flüssigkeit und der zweiten Flüssigkeit durch die Rotation von dem Übergangsbereich (54, 154, 154a, 154j) weg bewegt wird.

2. Verfahren nach Anspruch 1, bei dem ein Fluidkanal (52, 152) verwendet wird, der in einem radial äußeren Bereich in die Fluidkammer (50, 150) mündet, und bei dem eine zweite Flüssigkeit verwendet wird, die eine höhere Dichte aufweist als die erste Flüssigkeit, und bei dem die zweite Flüssigkeit durch die auf sie wirkende Zentrifugalkraft an dem Übergangsbereich (54, 154, 154a, 154j) gehalten wird.

3. Verfahren nach einem der Ansprüche 1 oder 2, bei dem die erste Flüssigkeit eine biochemische Reaktionsmischung aufweist, die für eine Detektion der DNA oder RNA geeignet ist.

4. Verfahren nach Anspruch 3, das ferner ein Durchführen einer DNA-Nachweisreaktion oder RNA-Nachweisreaktion der biochemischen Reaktionsmischung und ein Auslesen eines Reaktionsergebnisses aufweist.

## Claims

1. Method for generating one or several drops of a first liquid in a second liquid immiscible with the first liquid by using an apparatus for generating one or several drops of a first liquid in a second liquid immiscible with the first liquid, the apparatus comprising:
a rotational body (10) comprising fluidic structures, the fluidic structures comprising:
a fluid chamber (50, 150) configured to include the second liquid;
a fluid channel (52, 152) leading into the fluid chamber (50, 150) and configured to cause a flow of the first liquid in a flow direction to the fluid chamber (50, 150); and
a transition area (54, 154, 154a, 154j) where the fluid channel (52, 152) leads into the fluid chamber (50, 150), the transition area (54, 154, 154a, 154j) comprising a first expansion area (54a) where the flow cross-section for the flow of the first liquid expands in at least a first direction perpendicular to the flow direction and a second expansion area (54b) where the flow cross-section for the flow of the first liquid expands in a second direction perpendicular to the flow direction and to the first direction, the second expansion area (54b) being arranged downstream from the first expansion area (54a); and
a drive apparatus (22) configured to provide the rotational body (10) with such a rotation that the first liquid is supplied centrifugally to the fluid chamber (50, 150) and that centrifugally and hydrodynamically induced pressure, lifting and capillary forces are caused due to the second expansion area (54b), which cause a drop break-off in the first liquid, so that a drop of the first liquid is generated that is embedded in the second liquid,
wherein the apparatus is configured for generating a drop of the first liquid with a first density in a second liquid with a second density, wherein a) the first density is larger than the second density and the fluid channel (52, 152) leads into the fluid chamber (50, 150) in a radially inner area, or b) the second density is larger than the first density and the fluid channel (52, 152) leads into the fluid chamber (50, 150) in a radially outer area.
the method comprising:
rotating the rotational body (10) in order to supply the first liquid centrifugally to the fluid chamber (50, 150), into which the second liquid is introduced, through the fluid channel (52, 152) and to control, in the second expansion area, the centrifugally generated pressure force, lifting force and capillary force acting on the first liquid such that a drop break-off of the first liquid is caused, so that a drop of the first liquid is generated that is embedded in the second liquid,
wherein, after generating the drop, the drop is moved away from the transition area (54, 154, 154a, 154j) by the rotation due to different densities of the first liquid and the second liquid.

2. Method according to claim 1, wherein a fluid channel (52, 152) is used that leads into the fluid chamber (50, 150) in a radially outer area and wherein a second liquid with a higher density than the first liquid is used and wherein the second liquid is maintained at the transition area (54, 154, 154a, 154j) by the centrifugal force acting on the same.

3. Method according to one of claims 1 or 2, wherein the first liquid comprises a biochemical reaction mixture suitable for detecting the DNA or RNA.

4. Method according to claim 3, further comprising performing a DNA verification reaction or RNA verification reaction of the biochemical reaction mixture and reading out a reaction result.

## Revendications

1. Procédé pour générer une ou plusieurs gouttes d'un premier liquide dans un deuxième liquide non miscible avec le premier liquide à l'aide d'un dispositif pour générer une ou plusieurs gouttes d'un premier liquide dans un deuxième liquide non miscible avec le premier liquide, dans lequel le dispositif présente les caractéristiques suivantes:
un corps rotatif (10) qui présente des structures fluidiques, où les structures fluidiques présentent les caractéristiques suivantes:
une chambre à fluide (50, 150) qui est conçue pour contenir le deuxième liquide;
un canal à fluide (52, 152) qui débouche dans la chambre à fluide (50, 150) et est conçu pour provoquer une circulation du premier liquide dans une direction de circulation vers la chambre à fluide (50, 150); et
une zone de transition (54, 154, 154a, 154j) dans laquelle le canal à fluide (52, 152) débouche dans la chambre à fluide (50, 150), la zone de transition (54, 154, 154a, 154j) présentant une première zone d'élargissement (54a) dans laquelle la section transversale de circulation s'élargit pour la circulation du premier liquide dans au moins une première direction perpendiculaire à la direction de circulation, et présentant une deuxième zone d'élargissement (54b) dans laquelle la section transversale s'élargit pour la circulation du premier liquide dans une deuxième direction qui est perpendiculaire à la direction de circulation et à la première direction, la deuxième zone d'élargissement (54b) étant disposée en aval de la première zone d'élargissement (54a); et
un dispositif d'entraînement (22) qui est conçu pour soumettre le corps rotatif (10) à une rotation telle que le premier liquide soit alimenté de manière centrifuge vers la chambre à fluide (50, 150) et que du fait de la deuxième zone d'élargissement (54b) soient provoquées des forces de pression, d'élan et capillaires induites de manière centrifuge et hydrodynamique qui provoquent un arrachement de goutte dans le premier liquide, de sorte que soit générée une goutte du premier liquide noyée dans le deuxième liquide,
dans lequel le dispositif est conçu pour générer une goutte d'un premier liquide avec une première densité dans un deuxième liquide avec une deuxième densité, dans lequel a) la première densité est supérieure à la deuxième densité et le canal à fluide (52, 152) débouche dans une région radialement intérieure dans la chambre à fluide (50, 150), ou b) la deuxième densité est supérieure à la première densité et le canal de fluide (52, 152) débouche dans une zone radialement extérieure dans la chambre à fluide (50, 150),
dans lequel le procédé présente les caractéristiques suivantes consistant à:
faire tourner le corps rotatif (10) pour alimenter de manière centrifuge le premier liquide à travers le canal à fluide (52, 152) vers la chambre à fluide (50, 150) dans laquelle est introduit le deuxième liquide et pour contrôler, dans la deuxième zone d'élargissement, la force de pression, la force d'élan et la force capillaire générées de manière centrifuge agissant sur le premier liquide de sorte que soit provoqué un arrachement de goutte du premier liquide de sorte que soit générée une goutte du premier liquide noyée dans le deuxième liquide,
après la génération de la goutte, la goutte étant, par suite de différentes densités du premier liquide et du deuxième liquide, éloignée par rotation de la zone de transition (54, 154, 154a, 154j).

2. Procédé selon la revendication 1, dans lequel est utilisé un canal à fluide (52, 152) qui débouche dans la chambre à fluide (50, 150) dans une zone radialement extérieure, et dans lequel est utilisé un deuxième liquide qui présente une densité supérieure à celle du premier liquide, et dans lequel le deuxième liquide est maintenu par la force centrifuge agissant sur ce dernier dans la zone de transition (54, 154, 154a, 154j).

3. Procédé selon l'une des revendications 1 ou 2, dans lequel le premier liquide présente un mélange réactionnel biochimique qui convient pour la détection de l'ADN ou de l'ARN.

4. Procédé selon la revendication 3, présentant par ailleurs le fait d'effectuer une réaction de détection d'ADN ou une réaction de détection d'ARN du mélange réactionnel biochimique et de lire un résultat de réaction.
